# EUROPEAN PATENT SPECIFICATION

(11) **EP 1 351 780 B1**
(45) Date of publication and mention of the grant of the patent: **13.10.2004**
(21) Application number: 01987699.4
(22) Date of filing: 15.10.2001
(51) Int. Cl.: B05D 1/18, B05D 3/10

(54) **PROCESS FOR COATING A MATERIAL SURFACE**
VERFAHREN FÜR DIE OBERFLÄCHENBESCHICHTUNG VON MATERIALIEN
PROCEDE DE REVETEMENT DE LA SURFACE D'UN MATERIAU

(30) Priority: 16.10.2000 EP 00122542
(43) Date of publication of application: 15.10.2003
(73) Proprietor: Novartis AG, 4002 Basel (CH); Novartis Pharma GmbH, 1230 Wien (AT)
(72) Inventor: LEUKEL, Jörg, 79110 Freiburg (DE); CHABRECEK, Peter, CH-4125 Riehen (CH); LOHMANN, Dieter, CH-4142 Münchenstein (CH)
(74) Representative: Gros, Florent
(86) International application number: PCT/EP2001/011883
(87) International publication number: WO 2002/032590

(56) References cited:
- EP-A- 0 632 329
- WO-A-00/29130
- WO-A-94/06485
- WO-A-96/20796
- WO-A-96/20919
- WO-A-99/15917
- WO-A-99/57581
- US-A- 5 071 732

## Description

The present invention relates to a process for coating articles, wherein the coating comprises a polymer having desirable characteristics regarding adherence to the substrate, durability, softness, hydrophilicity, lubricity, wettability, biocompatibility and permeability. More particular, the present invention relates to a process for coating an article, such as a biomedical material or article, especially a contact lens including an extended-wear contact lens, wherein at least a part of the coating comprises a polymer having a "bottle-brush" type structure composed of tethered "hairy" chains. The inventive coatings are obtainable by grafting specific ethylenically unsaturated macromonomers onto the surface of a substrate, which has been previously provided with initiator groups.

A variety of different types of processes for preparing hydrophilic polymeric coatings on an "inert" hydrophobic substrate have been disclosed in the prior art. For example, WO 99/57581 discloses to first of all provide the article surface with covalently bound photoinitiator molecules, coating the modified surface with a layer of a polymerizable macromonomer and then subjecting it to a heat or radiation treatment whereby the macromonomer is graft polymerized thus forming the novel article surface. The covalent binding of the photoinitiator molecules to the article surface is created by first subjecting the article surface to a plasma treatment thereby providing the surface with functional groups, and then reacting said functional groups with co-reactive groups of a functional photoinitiator.

A plasma treatment requires a considerable investment in equipment and is furthermore difficult to be integrated in an automated production process. For example, a plasma treatment requires that the article to be treated is dry before exposure to the plasma. Thus, a polymeric article such as a contact lens that is wet from prior hydration or extraction must be dried previously, thereby adding time in the overall lens production process as well as imposing added costs of obtaining a drying equipment. Therefore, it would be highly desirable to modify the surface functionalization step of the process disclosed in WO 99/57581 such that the plasma treatment is avoided and replaced by a technique which is easy to perform with standard equipment and which is thus more feasible for an automated production process.

Surprisingly, it has now been found, that a large variety of articles may be readily functionalized by means of certain hetero-bifunctional compounds having a first highly reactive functional group, which is able to react with the "inert" article surface, and a second functional group for further covalent attachment of reactive molecules such as initiators, catalysts, polymers, enzymes and biocomponents.

The present invention therefore in one aspect relates to a process for coating a material surface comprising the steps of:
(a) reacting the material surface with a compound of formula 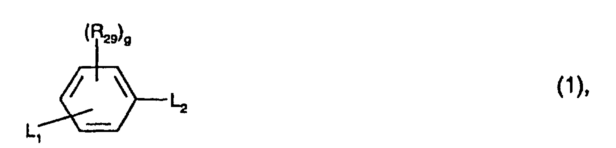 wherein R₂₉ is C₁-C₄-alkyl, C₁-C₄-alkoxy, amino, hydroxy, sulfo, nitro, trifluoromethyl or halogen,
   g is an integer from 0 to 2,
   L₁ is a group, which functions as a triggerable precursor for carbene, nitrene or benzhydrol formation,
   L₂ is amino, C₁-C₄-alkylamino, hydroxy, glycidyl, carboxy or a derivative thereof, isocyanato or isothiocyanato, or is a radical of formula

   - [L₃]ₕ - (spacer) - L₂' (1a),

   or L₂ and R₂₉ together form an anhydride radical 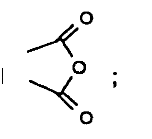 L₂' is amino, C₁-C₄-alkylamino, hydroxy, carboxy or a derivative thereof, isocyanato, isothiocyanato, -O-glycidyl or -O-C(O)-(CH₂)ₕ₁- X₂, wherein h1 is from 1 to 4 and X₂ is carboxy or a derivative thereof,
   L₃ is -NH-, -NC₁-C₆-alkyl-, -O-, -C(O)O-, -C(O)NH-, -NHC(O)NH-, -NHC(O)O- or - OC(O)NH-;
   (spacer) is linear or branched C₁-C₂₀₀-alkylene which may be substituted by hydroxy and/or interrupted by -O- except for C₁-alkyl, or is C₃-C₈-cycloalkylene, C₃-C₈-cycloalkylene-C₁-C₆-alkylene, C₃-C₈-cycloalkylene-C₁-C₂-alkylene-C₃-C₈-cycloalkylene or C₁-C₆-alkylene-C₃-C₈-cycloalkylene-C₁-C₆-alkylene; and
   h is the number 0 or 1;
(b) reacting the so modified surface with a functional polymerization initiator having a functional group that is co-reactive to L₂ or L₂'; and
(c) applying one or more different ethylenically unsaturated hydrophilic monomers or macromonomers to the bulk material surface obtainable according to step (b) and polymerizing said monomers or macromonomers, thereby providing a preferably hydrophilic surface coating onto the material surface.

Suitable materials to be coated according to the invention are, for example, natural or synthetic organic polymers, or laminates, composites or blends of said materials, in particular natural or synthetic organic polymers or modified biopolymers which are known in large number. Some examples of polymers are polyaddition and polycondensation polymers (polyurethanes, epoxy resins, polyethers, polyesters, polyamides and polyimides); vinyl polymers (polyacrylates, polymethacrylates, polyacrylamides, polymethacrylamides, polystyrene, polyethylene and halogenated derivatives thereof, polyvinyl acetate and polyacrylonitrile); or elastomers (silicones, polybutadiene and polyisoprene).

A preferred group of materials to be coated are those being conventionally used for the manufacture of biomedical devices, e.g. contact lenses, in particular contact lenses for extended wear, which are not hydrophilic per se. Such materials are known to the skilled artisan and may comprise for example polysiloxanes, perfluoroalkyl polyethers, fluorinated poly(meth)acrylates or equivalent fluorinated polymers derived e.g. from other polymerizable carboxylic acids, polyalkyl (meth)acrylates or equivalent alkylester polymers derived from other polymerizable carboxylic acids, or fluorinated polyolefines, such as fluorinated ethylene or propylene, for example tetrafluoroethylene, preferably in combination with specific dioxols, such as perfluoro-2,2-dimethyl-1,3-dioxol. Examples of suitable bulk materials are e.g. lotrafilcon A, neofocon, pasifocon, telefocon, silafocon, fluorsilfocon, paflufocon, elastofilcon, fluorofocon or teflon AF materials, such as teflon AF 1600 or teflon AF 2400 which are copolymers of about 63 to 73 mol % of perfluoro-2,2-dimethyl-1,3-dioxol and about 37 to 27 mol % of tetrafluoroethylene, or of about 80 to 90 mol % of perfluoro-2,2-dimethyl-1,3-dioxol and about 20 to 10 mol % of tetrafluoroethylene.

Another group of preferred materials to be coated are amphiphilic segmented copolymers comprising at least one hydrophobic segment and at least one hydrophilic segment which are linked through a bond or a bridge member. Examples are silicone hydrogels, for example those disclosed in PCT applications WO 96/31792 and WO 97/49740 which are herewith incorporated by reference.

A particular preferred group of materials to be coated comprises organic polymers selected from polyacrylates, polymethacrylates, polyacrylamides, poly(N,N-dimethylacrylamides), polymethacrylamides, polyvinyl acetates, polysiloxanes, perfluoroalkyl polyethers, fluorinated polyacrylates or -methacrylates and amphiphilic segmented copolymers comprising at least one hydrophobic segment, for example a polysiloxane or perfluoroalkyl polyether segment or a mixed polysiloxane/perfluoroalkyl polyether segment, and at least one hydrophilic segment, for example a polyoxazoline, poly(2-hydroxyethylmethacrylate), polyacrylamide, poly(N,N-dimethylacrylamide), polyvinylpyrrolidone polyacrylic or polymethacrylic acid segment or a copolymeric mixture of two or more of the underlying monomers.

The material to be coated may also be any blood-contacting material conventionally used for the manufacture of renal dialysis membranes, blood storage bags, pacemaker leads or vascular grafts. For example, the material to be modified on its surface may be a polyurethane, polydimethylsiloxane, polytetrafluoroethylene, polyvinylchloride, Dacron™ or Silastic™ type polymer, or a composite made therefrom.

The form of the material to be coated may vary within wide limits. Examples are particles, granules, capsules, fibres, tubes, films or membranes, preferably moldings of all kinds such as ophthalmic moldings, for example intraocular lenses, artificial cornea or in particular contact lenses.

L₁ in formula (1) is, for example, a group of formula

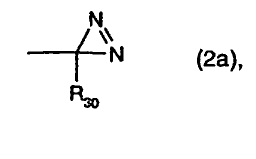

-N₃ (2b),

or 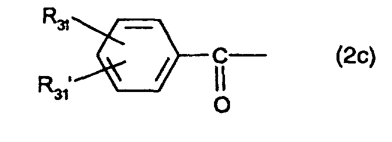 wherein R₃₀ is an electron-withdrawing substituent, for example fluorinated C₁-C₆-alkyl, such as a radical -C₂F₅ or preferably a radical -CF₃, and R₃₁ and R₃₁' are each independently of the other hydrogen, amino, hydroxy, glycidyl, -O-(CH₂)₂₋₄-O-glycidyl, carboxy, a carboxy derivative or isocyanato, or R₃₁ and R₃₁' together are an anhydride radical

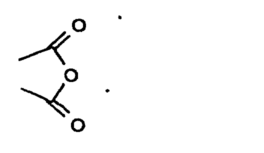

R₂₉ is preferably C₁-C₄-alkoxy, nitro, C₁-C₄-alkyl, hydroxy, amino or sulfo. The variable g is, for example, 1 or preferably 0.

R₃₁ is preferably hydrogen or amino and R₃₁' is preferably hydrogen; a further preferred embodiment relates to a radical of formula (2c), wherein R₃₁ and R₃₁' together are an anhydride radical as outlined above.

One group of suitable radicals of formula (1) are those wherein L₁ is a group 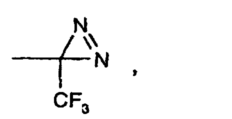 and g is 0. A further group of suitable radicals of formula (1) are those wherein L₁ is a group -N₃, and g is 1 or preferably 0. Still a further group of suitable radicals of formula (1) are those, wherein L₁ is a group of formula (2c) above, and wherein R₃₁ is hydrogen or amino and R₃₁' is hydrogen, or R₃₁ and R₃₁' together are an anhydride radical

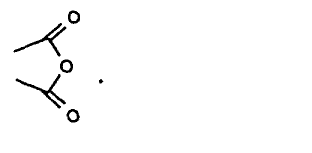

Throughout the application the terms carboxy derivative, a derivative of carboxy and the like are to be understood as meaning, for example, a lactone, a carboxylic acid anhydride, halide, amide or ester, for example -C(O)Cl, -C(O)NH₂, -C(O)C₁-C₆-alkyl, -C(O)-phenyl or in particular an activated ester such as carboxy having been reacted with an activating agent, for example with N-hydroxy succinimide (NHS) or sulfo-N-hydroxy succinimide. A particularly preferred carboxy derivative is an activated ester of formula

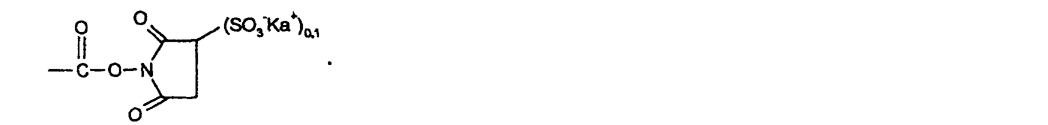

The term glycidyl means a radical 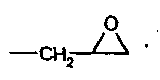 The bivalent radcals L₃ are always to be understood that the left bond is directed to the phenyl ring and the right bond is directed to the (spacer) radical.

According to one preferred embodiment of the invention, L₂ is amino, isocyanato, isothiocyanato, carboxy or a derivative thereof, and in particular amino, isocyanato, carboxy, or an activated carboxylic acid ester as mentioned above.

L₃ in formula (1a) is preferably a bivalent group -O-, -NH-, -C(O)O-, -C(O)NH- or - NHC(O)NH-, and is most preferably a radicat -NH-, -C(O)O- or -C(O)NH-. h is preferably the number 1.

(spacer) in formula (1a) is preferably linear or branched, optional hydroxy-substituted, C₁-C₂₄-alkylene or C₄-C₁₆₀-alkylene which is interrupted by -O-, more preferably C₁-C₁₆-alkylene or C₈-C₁₆₀-alkylene which is interrupted by -O- and most preferably C₂-C₁₂-alkylene or - (alk')-O-(CH₂CH₂O)₁₈₋₁₆₀-(alk')-, wherein (alk') is, for example, C₁-C₆-alkylene, preferably C₁-C₄-alkylene, more preferably C₁-C₃-alkylene and in particular 1,2-ethylene. If (spacer) is a cycloalkylene or mixed alkylene/cycloalkylene radical, the meanings and preferences given below for R₃₃ apply.

L₂' is preferably amino, isocyanato, carboxy, a carboxy derivative, or a radical -O-C(O)-(CH₂)₂-X₂, wherein X₂ is carboxy or a derivative thereof. Particularly preferred meanings of L₂' are amino, carboxy and an activated carboxylic acid ester as mentioned above.

A further preferred embodiment of the invention relates to the use of a compound of formula (1), wherein L₂ is a radical of formula (1a), L₃ is -NH-, -C(O)O- or -C(O)NH-, h is 1, (spacer) is linear C₂-C₁₂-alkylene or -(C₂-C₃-alkylene)-O-(CH₂CH₂O)₁₈₋₁₆₀-(C₂-C₃-alkylene)-, and L₂' is carboxy, a carboxy derivative or a radical -O-C(O)-(CH₂)₂-X₂, wherein X₂ is carboxy or an activated carboxylic acid ester as mentioned above.

Preferably, L₁ is a group of formula 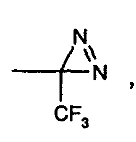 g is 0, and L₂ is carboxy, a carboxy derivative, or a radical of formula (1a) above, wherein the above-given meanings and preferences apply.

According to another preferred embodiment, L₁ is a group -N₃, g is 1 or preferably 0, R₂₉ is methyl, methoxy, hydroxy or nitro, and L₂ is amino, carboxy, a carboxy derivative, isocyanato, isothiocyanato or a radical of formula (1a) above, wherein the above-mentioned meanings and preferences apply, in particular amino.

According to still a further preferred embodiment, L₁ is a radical of formula (2c) above, wherein R₃₁ is hydrogen or amino and R₃₁' is hydrogen, or R₃₁ and R₃₁' together are a radical 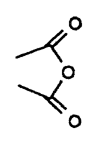 and L₂ is amino, g is 0 or 1 and R₂₉ is amino, or L₂ and R₂₉ together are a radical

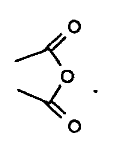

The compounds of formula (1) may be applied to the material surface according to processes known per se. For example, the bulk material is immersed in a solution of a compound of formula (1), or a layer of a compound of formula (1) is first of all deposited on the bulk material surface to be modified, for example, by dipping, spraying, printing, spreading, pouring, rolling, spin coating or vacuum vapor deposition, with dipping or spraying being preferred. Most preferably, a solution comprising one or more different compounds of the formula (1) is sprayed onto the bulk material surface, which may be dry or preferably wet. The compound of formula (1) may be applied to the material surface in one cycle or in repeated cycles.

Suitable solvents useful as solvents of the compounds of formula (1) are, for example, water, C₁-C₄-alkanols such as methanol, ethanol or iso-propanol, nitriles such. as acetonitrile, tetrahydrofurane (THF), aqueous solutions comprising an alkanol, THF or the like, ketones, for example acetone or methylethyl ketone, and also hydrocarbons, for example halogenated hydrocarbons such as methylene chloride or chloroform. The concentration of the compound of formula (1) in the spray solution depends on the specific compound used but is in general in the range of from 0.1 to 100 g/l, preferably 0.5 to 50 g/l, more preferably 0.5 to 25 g/l and in particular 1 to 10 g/l.

The fixation of the compounds of formula (1) on the bulk material surface then may be initiated, for example, by irradiation, particularly by irradiation with UV or visible light. Suitable light sources for the irradiation are known to the artisan and comprise for example mercury lamps, high pressure mercury lamps, xenon lamps, carbon arc lamps or sunlight. Sensitizers may be used to shift the irradiation wavelength. In addition, a suitable filter may be used to limit the irradiation to a specific wavelength range. Preferably, the bulk material surface to which the compound(s) of formula (1) have been previously applied, is irradiated with light of a wavelength ≥250nm and preferably ≥300nm. The time period of irradiation is not critical but is usually in the range of up to 30 minutes, preferably from 10 secondes to 10 minutes, and more preferably from 15 seconds to 5 minutes, and particularly preferably from 20 seconds to 1 minute. The irradiation may be carried out under ambient conditions or in an atmosphere of inert gas. Masks can be used for the generation of specific surface patterns of functional groups. Following the fixation reaction, any non-covalently bound compounds can be removed, for example by treatment, e.g. extraction, with suitable solvents, for example water, C₁-C₄-alkanols, water/C₁-C₄-alkanol mixtures or acetonitrile.

Depending on the desired concentration of functional groups L₂ on the material surface, the above outlined process cycle, (i) contacting, i.e. spraying or dipping, the surface with the compound(s) of formula (1) and (ii) fixing the compound(s) of formula (1) on the surface, i.e. by irradiation, may be carried out once or, preferably, several times. For example, 1 to 100, preferably 1 to 50 and in particular 5 to 25, different layers of one or more compounds of formula (1) are added and fixed on the material surface.

A polymerization initiator according to step (b) is typically one that is initiating a radical polymerization of ethylenically unsaturated compounds. The radical polymerization may be induced thermally, or preferably by irradiation.

Suitable thermal polymerization initiators are known to the skilled artisan and comprise for example peroxides, hydroperoxides, azo-bis(alkyl- or cycloalkylnitriles), persulfates, percarbonates or mixtures thereof. Examples are benzoylperoxide, tert.-butyl peroxide, di-tert.-butyl-diperoxyphthalate, tert.-butyl hydroperoxide, azo-bis(isobutyronitrile), 1,1'-azo-bis (1-cyclohexanecarbonitrile), 2,2'-azo-bis(2,4-dimethylvaleronitrile), 4,4'-azo-bis(4-cyano-valeric acid, 4,4'-azo-bis(4-cyano-n-pentanol) and the like. Initiators for the thermal polymerization are particularly functional initiators having an initiator part such as a peroxide, hydroperoxide, persulfate or azo group and in addition a functional group that is co-reactive with the functional groups L₂ of the modified material surface obtainable according to step (a). Suitable functional groups that are co-reactive with L₂ are, for example, a carboxy, amino, hydroxy, epoxy or isocyanato group. A particular preferred group of thermal initiators are azo-bis(C₂-C₁₂-alkane carboxylic acids) or azo-bis(C₂-C₁₂-alkanols) wherein the alkane moiety in each case may be further substituted, for example, by cyano.

Initiators for the radiation-induced polymerization are particularly functional photoinitiators having a photoinitiator part and in addition a functional group that is co-reactive with the functional groups L₂ of the modified material surface obtainable according to step (a). The photoinitiator part may belong to different types, for example to the thioxanthone type and preferably to the benzoin type. Suitable functional groups that are co-reactive with L₂ are, for example, a carboxy, amino, hydroxy, epoxy or isocyanato group.

Preferred polymerization initiators for use in the present invention are the photoinitiators of formulae (I) and (Ia) as disclosed in US patent No. 5,527,925, those of the formula (I) as disclosed in PCT application WO 96/20919, or those of formulae II and III including formulae IIa-IIy and IIIg as disclosed in EP-A-0281941, particularly formulae IIb, IIi, IIm, IIn, IIp, IIr, IIs, IIx and IIIg therein. The respective portion of said three documents including the definitions and preferences given for the variables in said formulae are herewith included by reference.

The polymerization initiator moieties are preferably derived from a functional photoinitiator of the formula 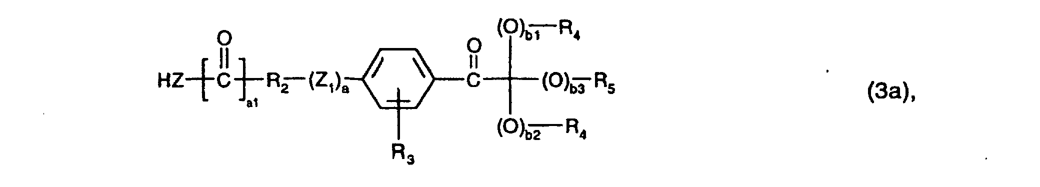 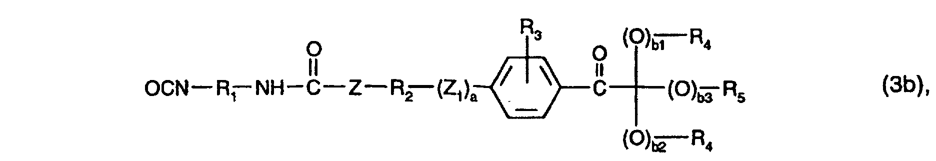 or 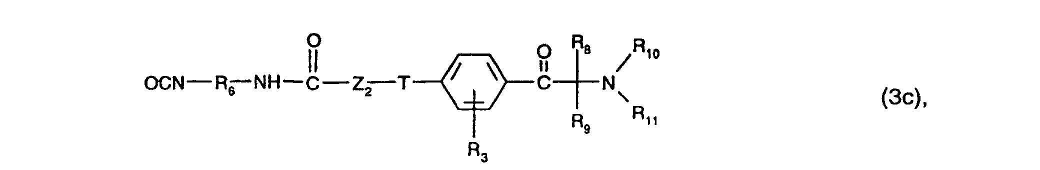 wherein Z is bivalent -O-, -NH- or -NR₁₂-; Z₁ is -O-, -O-(O)C-, -C(O)-O- or -O-C(O)-O-; R₃ is H, C₁-C₁₂-alkyl, C₁-C₁₂-alkoxy or N-C₁-C₁₂-alkylamino; R₄ and R₅ are each independently of the other H, linear or branched C₁-C₈-alkyl, C₁-C₈-hydroxyalkyl or C₆-C₁₀-aryl, or the groups R₄-(O)_{b1}- and R₄-(O)_{b2}- together are -(CH₂)_{c}- wherein c is an integer from 3 to 5, or the groups R₄-(O)_{b1}-, R₄-(O)_{b2}- and R₅-(O₁)_{b3}- together are a radical of the formula 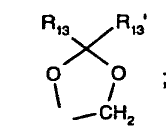 R₂ is a direct bond or linear or branched C₁-C₈-alkylene that is unsubstituted or substituted by -OH and/or is uninterrupted or interrupted by one or more groups -O-, - O-C(O)- or -O-C(O)-O-; R₁ is branched C₃-C₁₈-alkylene, unsubstituted or C₁-C₄-alkyl- or C₁-C₄-alkoxy-substituted C₆-C₁₀-arylene, or unsubstituted or C₁-C₄-alkyl- or C₁-C₄-alkoxy-substituted C₇-C₁₈-aralkylene, unsubstituted or C₁-C₄-alkyl- or C₁-C₄-alkoxy-substituted C₃-C₈-cycloalkylene, unsubstituted or C₁-C₄-alkyl- or C₁-C₄-alkoxy-substituted C₃-C₈-cyclo-alkylene-C_{y}H_{2y}- or unsubstituted or C₁-C₄-alkyl- or C₁-C₄-alkoxy-substituted -C_{y}H_{2y}-(C₃-C₈-cycloalkylene)-C_{y}H_{2y}- wherein y is an integer from 1 to 6; R₆ independently has the same definitions as R₁ or is linear C₃-C₁₈-alkylene; R₁₂ is linear or branched C₁-C₆-alkyl; T is bivalent -O-, -NH-, -S-, C₁-C₈-alkylene or 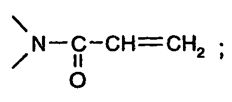 Z₂ is a direct bond or - O-(CH₂)_{d}- or -(OCH₂CH₂)_{d}- wherein d is an integer from 1 to 6 and the terminal CH₂ group of which is each linked to the adjacent T in formula (3c); R₈ is linear or branched C₁-C₈-alkyl, C₂-C₈-alkenyl or C₆-C₁₀-aryl-C₁-C₈-alkyl; R₉ independently of R₈ has the same definitions as R₈ or is C₆-C₁₀-aryl, or R₈ and R₉ together are -(CH₂)ₑ- wherein e is an integer from 2 to 6; R₁₀ and R₁₁ are each independently of the other linear or branched C₁-C₈-alkyl that may be substituted by C₁-C₄-alkoxy, or C₆-C₁₀-aryl-C₁-C₈-alkyl or C₂-C₈-alkenyl; or R₁₀ and R₁₁ together are -(CH₂)_{f1}-Z₃-(CH₂)_{f2}- wherein Z₃ is a direct bond, -O-, -S- or -NRr₇ and R₇ is H or C₁-C₈-alkyl and f1 and f2 are each independently of the other an integer from 2 to 4; R₁₃ and R₁₃' are each independently of the other H, C₁-C₈-alkyl, C₃-C₈-cycloalkyl, benzyl or phenyl; and a, a1, b1, b2 and b3 are each independently of the other 0 or 1; subject to the provisos that b1 and b2 are each 0 when R₁₅ is H; that the total of (b1+b2+b3) is not exceeding 2; and that a is 0 when R₁₂ is a direct bond.

A preferred sub-group of compounds of formula (3a) or (3b) comprises those wherein, b1 and b2 are each 0; Z and Z₁ are each bivalent -O-; b3 is 0 or 1; R₄ is C₁-C₄-alkyl or phenyl, or both groups R₄ together are tetramethylene or pentamethylene; R₅ is C₁-C₄-alkyl or H, R₃ is hydrogen; a and a1 are each independently 0 or 1; R₂ is linear or branched C₂-C₄-alkylene, or is a direct bond, in which case a is 0; R₁ is branched C₅-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexyl-C_{y}H_{2y}- or -C_{y}H_{2y}-cyclohexyl-C_{y}H_{2y}- or cyclohexyl-C_{y}H_{2y}- or -C_{y}H_{2y}-cyclohexyl-C_{y}H_{2y}- substituted by from 1 to 3 methyl groups; and y is 1 or 2.

An especially preferred sub-group of compounds of formula (3a) or (3b) comprises those wherein, b1 and b2 are each 0, Z and Z₁ are each bivalent -O-, b3 is 0 or 1; R₄ is methyl or phenyl, or both groups R₄ together are pentamethylene; R₅ is methyl or H; R₃ is hydrogen; a is 1 and R₂ is ethylene, or a is 0 and R₂ is a direct bond; a1 is 0 or 1; and R₁ is branched C₆-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexyl-CH₂- or cyclohexyl-CH₂- substituted by from 1 to 3 methyl groups.

A preferred sub-group of compounds of formula (3c) comprises those wherein T is bivalent -O-, -NH-, -S- or -(CH₂)_{y}- wherein y is an integer from 1 to 6; Z₂ is a direct bond or -O-(CH₂)_{y}- wherein y is an integer from 1 to 6 and the terminal CH₂ group of which is linked to the adjacent T in formula (3c); R₃ is H, C₁-C₁₂-alkyl or C₁-C₁₂-alkoxy; R₈ is linear C₁-C₈-alkyl, C₂-C₈-alkenyl or C₆-C₁₀-aryl-C₁-C₈-alkyl; R₉ independently of R₈ has the same definitions as R₈ or is C₆-C₁₀-aryl, or R₈ and R₉ together are -(CH₂)ₑ- wherein e is an integer from 2 to 6; R₁₀ and R₁₁ are each independently of the other linear or branched C₁-C₈-alkyl that may be substituted by C₁-C₄-alkoxy, or C₆-C₁₀-aryl-C₁-C₈-alkyl or C₂-C₈-alkenyl; or R₁₀ and R₁₁ together are -(CH₂)_{f1}-Z₃-(CH₂)_{f2}- wherein Z₃ is a direct bond, -O-, -S- or -NR₇-, and R₇ is H or C₁-C₈-alkyl and f1 and f2 are each independently of the other an integer from 2 to 4; and R₆ is branched C₆-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohexylene-CH₂- or cyclohexylene-CH₂- substituted by from 1 to 3 methyl groups.

An especially preferred sub-group of compounds of formula (3c) comprises those wherein T is bivalent -O-; Z₂ is -O-(CH₂)_{y}- wherein y is an integer from 1 to 4 and the terminal CH₂ group of which is linked to the adjacent T in formula (3c); R₃ is H; R₈ is methyl, allyl, tolylmethyl or benzyl, R₉ is methyl, ethyl, benzyl or phenyl, or R₈ and R₉ together are pentamethylene, R₁₀ and R₁₁ are each independently of the other C₁-C₄-alkyl or R₁₀ and R₁₁ together are -CH₂CH₂OCH₂CH₂-, and R₆ is branched C₆-C₁₀-alkylene, phenylene or phenylene substituted by from 1 to 3 methyl groups, benzylene or benzylene substituted by from 1 to 3 methyl groups, cyclohexylene or cyclohexylene substituted by from 1 to 3 methyl groups, cyclohoxylene-CH₂- or cyclohexylene-CH₂- substituted by from 1 to 3 methyl groups.

Some examples of especially preferred functional photoinitiators are the compounds of formulae 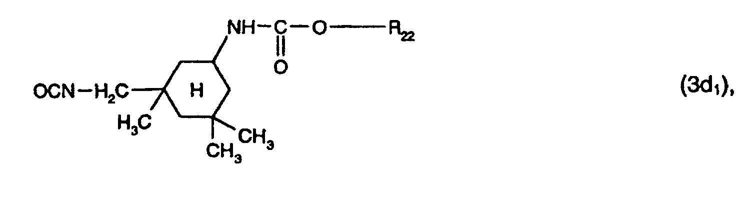 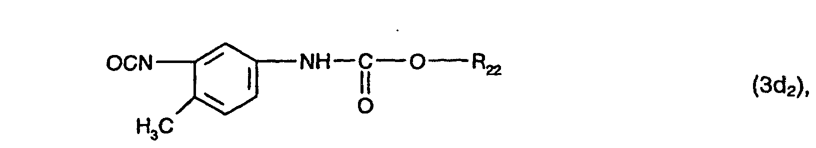 or

OCN-CH₂-C(CH₃)₂-CH₂-CH(CH₃)-CH₂-CH₂-NH-C(O)-O-R₂₂ (3d₃),

wherein R₂₂ is a radical 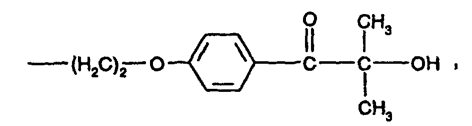 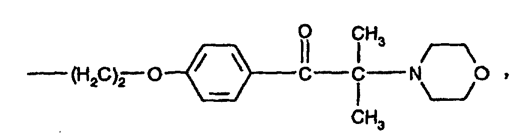 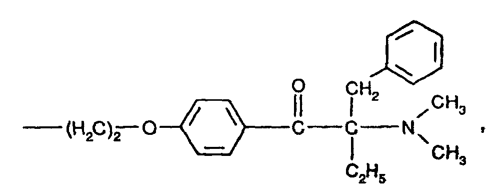 or

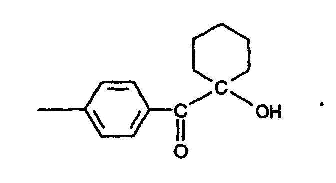

The reactions of radicals on the material surface that are derived from a compound of formula (1) having a carboxy, carboxy derivative, isocyanato or isothiocyanato group L₂ with a functional polymerisation initiator having an amino or hydroxy group, or vice versa, are well-known in the art and may be carried out as desribed in textbooks of organic chemistry. For example, the reaction of a radical derived from a compound of formula (1), wherein L₂ is an isocyanato or isothiocyanato group with an amino- or hydroxy-functionalized polymerisation initiator, or vice versa the reaction of an amino- or hydroxy group L₂ with an isocyanato or isothiocyanato functionalized polymerisation initiator, may be carried out in an inert organic solvent such as an optionally halogenated hydrocarbon, for example petroleum ether, methylcyclohexane, toluene, chloroform, methylene chloride and the like, or an ether, for example diethyl ether, tetrahydrofurane, dioxane, or a more polar solvent such as DMSO, DMA, N-methylpyrrolidone or even a lower alcohol, at a temperature of from 0 to 100°C, preferably from 0 to 50°C and particularly preferably at room temperature, optionally in the presence of a catalyst, for example a tertiary amine such as triethylamine or tri-n-butylamine, 1,4-diazabicyclooctane, or a tin compound such as dibutyltin dilaurate or tin dioctanoate. It is advantageous to carry out the above reactions under an inert atmosphere, for example under a nitrogen or argon atmosphere.

In case that the radicals on the material surface are derived from a compound of formula (1) having a carboxy group L₂, the reaction of the carboxy group with an amino or hydroxy group functionalized photoinitiator, or vice versa the reaction of an amino or hydroxy group L₂ with a carboxy functionalized polymerisation initiator, may be carried out under the conditions that are customary for ester or amide formation, for example in an aprotic medium at a temperature from about room temperature to about 100°C. It is further preferred to carry out the esterification or amidation reaction in the presence of an activating agent, for example N-ethyl-N'-(3-dimethylaminopropyl)carbodiimide (EDC), N-hydroxy succinimide (NHS), sulfo-N-hydroxy succinimide or N,N'-dicyclohexyl carbodiimide (DCC) or in the presence of an o-(benztriazole)- uronium salt such as o-(benztriazol-1-y-)-N,N,N,N-tetramethyluronium hexafluorophosphate. Most preferably, the carboxy group L₂ is previously converted to an activated ester using one of the above-mentioned activating agents, and the activated ester is then further reacted with the hydroxy or preferably amino groups of the surface.

In a preferred embodiment of the invention, L₂ comprises amino, alkylamino or hydroxy, particularly amino, as reactive group and the co-reactive group of the polymerization initiator is an isocyanato group. A preferred polymerization initiator of this embodiment is a photoinitiator of the above formula (3b), (3c), (3d₁), (3d₂) or (3d₃).

According to another preferred embodiment of the invention, L₂ comprises carboxy, a carboxy derivative, isocyanato or isothiocyanato as reactive group, and the co-reactive group of the polymerization initiator is a hydroxy, amino, alkylamino or thiol group, particularly an amino group. A preferred polymerization initiator of this embodiment is a photoinitiator of the above formula (3a).

A hydrophilic monomer useful to provide the hydrophilic surface coating (c) on the initiator-modified bulk material surface is typical a monomer that yields as homopolymer a polymer that is water-soluble or can absorb at least 10 % by weight of water. Examples of preferred hydrophilic monomers are hydroxy-substituted C₂-C₄-alkyl acrylates and methacrylates, acrylamide, methacrylamide, N,N-di-C₁-C₄-alkyl acrylamides and methacrylamides, ethoxylated acrylates and methacrylates, hydroxy-substituted C₂-C₄-alkyl acrylamides and methacrylamides, hydroxy-substituted C₁-C₄-alkyl vinyl ethers, sodium ethylenesulfonate, sodium styrenesulfonate, 2-acrylamido-2-methylpropanesulfonic acid, N-vinylpyrrole, N-vinylsuccinimide, N-vinylpyrrolidone, 2- or 4-vinylpyridine, acrylic acid, methacrylic acid, amino- (the term "arnino" also including quaternary ammonium), mono-C₁-C₄-alkylamino- or di-C₁-C₄-alkylamino-C₁-C₄-alkyl acrylates and methacrylates, allylalcohol and the like. Hydroxy-substituted or N,N-di-C₁-C₂-alkylamino-substituted C₂-C₄alkyl(meth)acrylates, five- to seven-membered N-vinyl lactams, N,N-di-C₁-C₄alkyl(meth)acrylamides and vinylically unsaturated carboxylic acids having a total of from 3 to 5 carbon atoms, for example, are preferred.

Examples of preferred hydrophilic vinylic monomers include hydroxyethyl methacrylate, hydroxyethyl acrylate, acrylamide, methacrylamide, N,N-dimethylacrylamide, allyl alcohol, N-vinylpyrrolidone, acrylic acid, methacrylic acid and N,N-dimethylaminoethyl methacrylate.

Preferably the hydrophilic surface coating (c) on the bulk material is obtained using a suitable macromonomer. A suitable macromonomer according to step (c) of the process of the invention is, for example, of formula 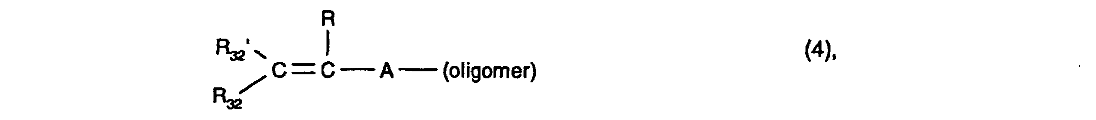 wherein R₃₂ is hydrogen, C₁-C₆-alkyl or a radical -COOR';
R, R' and R₃₂' are each independently of the other hydrogen or C₁-C₆-alkyl;
A is a direct bond or is a radical of formula

- C(O)-(A₁)ₙ-X- (5a)

or

- (A₂)ₘ-NH-C(O)-X- (5b);

or

- (A₂)ₘ-X-C(O)- (5c);

or

- C(O)-NH-C(O)-X- (5d);

or

- C(O)-X₁-(alk*)-X-C(O)- (5e);

or

A and R₃₂, together with the adjacent double bond, are a radical of formula 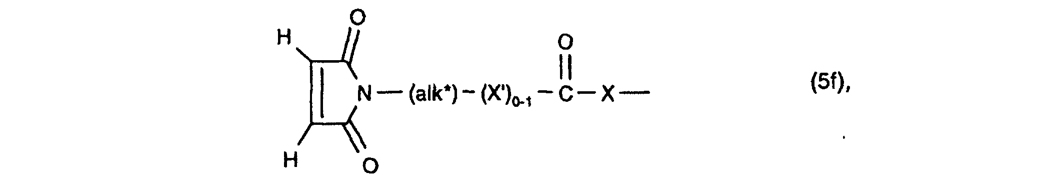 A₁ is -O-C₂-C₁₂-alkylene which is unsubstituted or substituted by hydroxy, or is - O-C₂-C₁₂-alkylene-NH-C(O)- or -O-C₂-C₁₂-alkylene-O-C(O)-NH-R₃₃-NH-C(O)- or -NH-(Alk*)-C(O)-, wherein (Alk*) is C₁-C₆-alkylene and R₃₃ is linear or branched C₁-C₁₈-alkylene or unsubstituted or C₁-C₄-alkyl- or C₁-C₄-alkoxy-substituted C₆-C₁₀-arylene, C₇-C₁₈-aralkylene, C₆-C₁₀-arylene-C₁-C₂-alkylene-C₆-C₁₀-arylene, C₃-C₈-cycloalkylene, C₃-C₈-cycloalkylene-C₁-C₆-alkylene, C₃-C₈-cycloalkylene-C₁-C₂-alkylene-C₃-C₈-cycloalkylene or C₁-C₆-alkylene-C₃-C₈-cycloalkylene-C₁-C₆-alkylene ;
A₂ is C₁-C₈-alkylene; phenylene or benzylene;
m and n are each independently of the other the number 0 or 1;
X, X₁ and X' are each independently of the other a bivalent group -O- or -NR", wherein R" is hydrogen or C₁-C₆-alkyl;
(alk*) is C₂-C₁₂-alkylene;
and (oligomer) denotes
(i) the radical of a telomer of formula 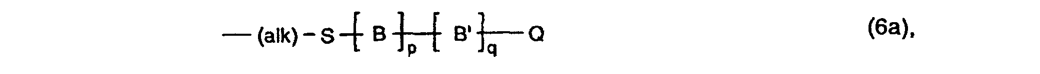 wherein (alk) is C₂-C₁₂-alkylene,
Q is a monovalent group that is suitable to act as a polymerization chain-reaction terminator,
p and q are each independently of another an integer from 0 to 350, wherein the total of (p+q) is an integer from 2 to 350,
and B and B' are each independently of the other a 1,2-ethylene radical derivable from a copolymerizable vinyl monomer by replacing the vinylic double bond by a single bond, at least one of the radicals B and B' being substituted by a hydrophilic substituent; or
(ii) the radical of an oligomer of the formula 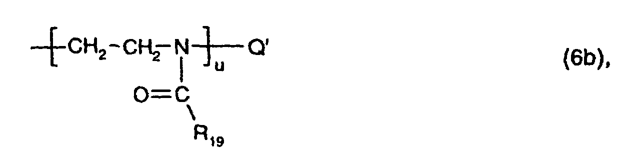 wherein R₁₉ is hydrogen or unsubstituted or hydroxy-substituted C₁-C₁₂-alkyl, u is an integer from 2 to 250 and Q' is a radical of a polymerization initiator; or
(iii) the radical of formula 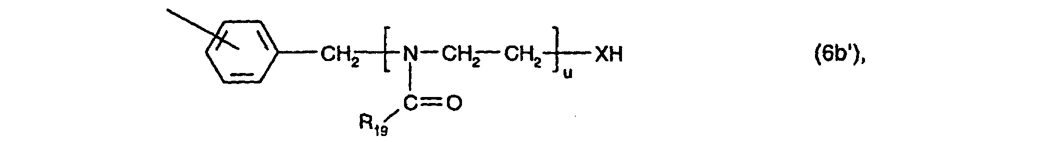 wherein R₁₉, X and u are as defined above, or
(iv) the radical of an oligomer of formula 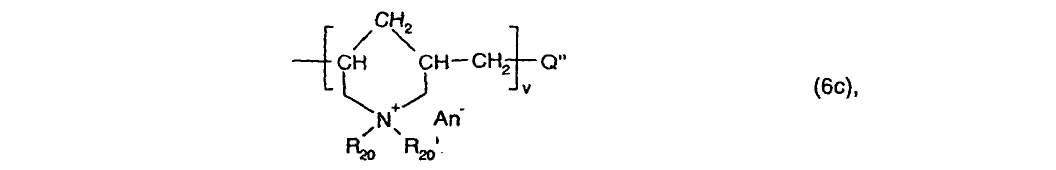 wherein R₂₀ and R₂₀' are each independently C₁-C₄-alkyl, An⁻ is an anion, v is an integer from 2 to 250, and Q" is a monovalent group that is suitable to act as a polymerization chain-reaction terminator; or
(v) the radical of an oligopeptide of formula

- (CHR₂₁-C(O)-NH)ₜ-CHR₂₁-COOH (6d)

or

- CHR₂₁-(NH-C(O)-CHR₂₁)ₜ-NH₂ (6d'),

wherein R₂₁ is hydrogen or C₁-C₄-alkyl which is unsubstituted or substituted by hydroxy, carboxy, carbamoyl, amino, phenyl, o-, m- or p-hydroxyphenyl, imidazolyl, indolyl or a radical -NH-C(=NH)-NH₂ and t is an integer from 2 to 250, or the radical of an oligopeptide based on proline or hydroxyproline; or
(vi) the radical of a polyalkylene oxide of formula

- (alk"-O)_{z} -[CH₂-CH₂-O]ᵣ-[CH₂-CH(CH₃)-O]ₛ-R₃₄ (6e),

wherein R₃₄ is hydrogen or C₁-C₂₄-alkyl, (alk") is C₂-C₄-alkylene, z is 0 or 1, r and s are each independently an integer from 0 to 250 and the total of (r+s) is from 2 to 250; or
(vii) the radical of an oligosaccharide;
subject to the provisos that
A is not a direct bond if (oligomer) is a radical of formula (6a);
A is a radical of formula (5a), (5b) or (5d) or A and R₃₂, together with the adjacent double bond, are a radical of formula (5f) if (oligomer) is a radical of formula (6b), (6c), (6d) or (6e) or is the radical of an oligosaccharide;
A is a direct bond if (oligomer) is a radical of formula (6b'); and
A is a radical of formula (5c) or (5e) if (oligomer) is a radical of formula (6d').

The following preferences apply to the variables contained in the definition of the macromonomer of formula (4):
R' is preferably hydrogen or C₁-C₄-alkyl, more preferably hydrogen or C₁-C₂-alkyl and particularly preferably hydrogen.
R₃₂ is preferably hydrogen, methyl or carboxyl, and particularly preferably hydrogen.
R is preferably hydrogen or methyl.
X is preferably a bivalent group -O- or -NH-. X is particularly preferably the group -NH- if (oligomer) is a radical of formula (6a); (6c) or (6d), and is particularly preferably the group -O- if (oligomer) is a radical of formula (6b) or (6e) or is the radical of an oligosaccharide. X' is preferably -O- or -NH- and more preferably -NH-. X₁ is preferably -O- or -NH-.
R₃₃ as alkylene is preferably a linear or branched C₃-C₁₄-alkylene radical, more preferably a linear or branched C₄-C₁₂alkylene radical and most preferably a linear or branched C₆-C₁₀-alkylene radical.

When R₃₃ is arylene, it is, for example, naphthylene or especially phenylene, each of which may be substituted, for example, by C₁-C₄-alkyl or by C₁-C₄-alkoxy. Preferably, R₃₃ as arylene is 1,3- or 1 ,4-phenylene that is unsubstituted or substituted by C₁-C₄-alkyl or by C₁-C₄-alkoxy in the ortho-position to at least one linkage site.

R₃₃ as aralkylene is preferably naphthylalkylene and most preferably phenylalkylene. The alkylene group in aralkylene contains preferably from 1 to 12, more preferably from 1 to 6 and most preferably from 1 to 4 carbon atoms. Most preferably, the alkylene group in aralkylene is methylene or ethylene.

When R₃₃ is cycloalkylene, it is preferably C₅-C₆-cycloalkylene and most preferably cyclohexylene that is unsubstituted or substituted by methyl.

If R₃₃ is cycloalkylene-alkylene, it is preferably cyclopentylene-C₁-C₄-alkylene and especially cyclohexylene-C₁-C₄-alkylene, each unsubstituted or mono- or poly-substituted by C₁-C₄-alkyl, especially methyl. More preferably, the group cycloalkylene-alkylene is cyclohexylene-ethylene and, most preferably, cyclohexylene-methylene, each unsubstituted or substituted in the cyclohexylene radical by from 1 to 3 methyl groups.

When R₃₃ is alkylene-cycloalkylene-alkylene, it is preferably C₁-C₄-alkylene-cyclopentylene-C₁-C₄-alkylene and especially C₁-C₄-alkylene-cyclohexylene-C₁-C₄-alkylene, each unsubstituted or mono- or poly-substituted by C₁-C₄-alkyl, especially methyl. More preferably, the group alkylene-cycloalkylene-alkylene is ethylene-cyclohexylene-ethylene and, most preferably, is methylene-cyclohexylene-methylene, each unsubstituted or substituted in the cyclohexylene radical by from 1 to 3 methyl groups.

R₃₃ as C₃-C₈-cycloalkylene-C₁-C₂-alkylene-C₃-C₈-cycloalkylene or C₆-C₁₀-arylene-C₁-C₂-alkylene-C₆-C₁₀-arylene is preferably C₅-C₆-cycloalkylene-methylene-C₅-C₆-cycloalkylene or phenylene-methylene-phenylene, each of which may be unsubstituted or substituted in the cycloalkyl or phenyl ring by one or more methyl groups.

The radical R₃₃ has a symmetrical or, preferably, an asymmetrical structure. A preferred group of radicals R₁₁ comprises those, wherein R₃₃ is linear or branched C₆-C₁₀alkylene; cyclohexylene-methylene or cyclohexylene-methylene-cyclohexylene each unsubstituted or substituted in the cyclohexyl moiety by from 1 to 3 methyl groups; or phenylene or phenylene-methylene-phenylene each unsubstituted or substituted in the phenyl moiety by methyl. The bivalent radical R₃₃ is derived preferably from a diisocyanate and most preferably from a diisocyanate selected from the group isophorone diisocyanate (IPDI), toluylene-2,4-diisocyanate (TDI), 4,4'-methylenebis(cyclohexyl isocyanate), 1,6-diisocyanato-2,2,4-trimethyl-n-hexane (TMDI), methylenebis(phenyl isocyanate), methylenebis(cyclohexyl-4-isocyanate) and hexamethylene diisocyanate (HMDI).

Preferred meanings of A₁ are unsubstituted or hydroxy-substituted -O-C₂-C₈-alkylene or a radical -O-C₂-C₆-alkylene-NH-C(O)- and particularly -O-(CH₂)₂₋₄-, -O-CH₂-CH(OH)-CH₂- or a radical -O-(CH₂)₂₋₄-NH-C(O)-. A particularly preferred meaning of A, is the radical - O-(CH₂)₂-NH-C(O)-.

A₂ is preferably C₁-C₆-alkylene, phenylene or benzylene, more preferably C₁-C₄-alkylene and even more preferably C₁-C₂-alkylene.
n is an integer of 0 or preferably 1. m is preferably an integer of 1.
R₃₂' is preferably hydrogen or methyl and particularly preferably hydrogen.
In case that (oligomer) is a radical of formula (6a), (6b), (6c), (6d) or (6e) or is the radical of an oligosaccharide, is A preferably a radical of formula (5a) or (5b) and particularly preferably a radical of formula (5a), wherein the above given meanings and preferences apply for the variables contained therein.
A preferred group of hydrophilic macromonomers according to the invention comprises compounds of the above formula (4), wherein R is hydrogen or methyl, R₃₂ is hydrogen, methyl or carboxyl, R₃₂' is hydrogen, A is a radical of the formula (5a) or (5b) and (oligomer) is a radical of formula (6a), (6b), (6c), (6d) or (6e) or is the radical of an oligosaccharide. An even more preferred group of hydrophilic macromonomers comprises compounds of the above formula (4), wherein R is hydrogen or methyl, R₃₂ and R₃₂' are each hydrogen, A is a radical of the formula (5a) and (oligomer) is a radical of formula (6a). A further group of preferred macromonomers comprises compounds of formula (4), wherein A is a radical of formula (5e) above and (oligomer) is a radical of formula (6a).

(Alk*) is preferably methylene, ethylene or 1,1-dimethyl-methylene, in particular a radical -CH₂- or -C(CH₃)₂-.

(alk) and (alk*) are each independently preferably C₂-C₈-alkylene, more preferably C₂-C₆-alkylene, even more preferably C₂-C₄-alkylene and particularly preferably 1,2-ethylene. The alkylene radicals (alk) and (alk*) may be branched or preferably linear alkylene radicals.

Q is for example hydrogen.

The total of (p+q) is preferably an integer from 2 to 150, more preferably from 5 to 100, even more preferably from 5 to 75 and particularly preferably from 10 to 50. In a preferred embodiment of the invention q is 0 and p is an integer from 2 to 250, preferably from 2 to 150, more preferably from 5 to 100, even more preferably from 5 to 75 and particularly preferably from 10 to 50.

Suitable hydrophilic substituents of the radicals B or B' may be non-ionic, anionic, cationic or zwitterionic substituents. Accordingly, the telomer chain of formula (5a) that contains monomer units B and/or B' may be a charged chain containing anionic, cationic and/or zwitterionic groups or may be an uncharged chain. In addition, the telomer chain may comprise a copolymeric mixture of uncharged and charged units. The distribution of the charges within the telomer, if present, may be random or blockwise.

In one preferrred embodiment of the invention, the telomer radical of formula (6a) is composed solely of non-ionic monomer units B and/or B'. In another preferred embodiment of the invention, the telomer radical of formula (6a) is composed solely of ionic monomer units B and/or B', for example solely of cationic monomer units or solely of anionic monomer units. Still another preferred embodiment of the invention is directed to telomer radicals of formula (6a) comprising nonionic units B and ionic units B'.

Suitable non-ionic substituents of B or B' include for example a radical C₁-C₆-alkyl which is substituted by one or more same or different substituents selected from the group consisting of -OH, C₁-C₄-alkoxy and -NR₂₃R₂₃', wherein R₂₃ and R₂₃' are each independently of another hydrogen or unsubstituted or hydroxy-substituted C₁-C₆-alkyl or phenyl; phenyl which is substituted by hydroxy, C₁-C₄-alkoxy or -NR₂₃R₂₃', wherein R₂₃ and R₂₃' are as defined above; a radical -COOY, wherein Y is C₁-C₂₄-alkyl which is unsubstituted or substituted, for example, by hydroxy, C₁-C₄-alkoxy, -O-Si(CH₃)₃, -NR₂₃R₂₃' wherein R₂₃ and R₂₃' are as defined above, a radical -O-(CH₂CH₂O)₁₋₂₄-E wherein E is hydrogen or C₁-C₆-alkyl, or a radical -NH-C(O)-O-G, wherein -O-G is the radical of a saccharide with 1 to 8 sugar units or is a radical -O-(CH₂CH₂O)₁₋₂₄-E, wherein E is as defined above, or Y is C₅-C₈-cycloalkyl which is unsubstituted or substituted by C₁-C₄-alkyl or C₁-C₄-alkoxy, or is unsubstituted or C₁-C₄-alkyl- or C₁-C₄-alkoxy-substituted phenyl or C₇-C₁₂-aralkyl; -CONY₁Y₂ wherein Y₁ and Y₂ are each independently hydrogen, C₁-C₁₂-alkyl, which is unsubstituted or substituted for example by hydroxy, C₁-C₄-alkoxy or a radical -O-(CH₂CH₂O)₁₋₂₄-E wherein E is as defined above, or Y₁ and Y₂ together with the adjacent N-atom form a five- or six-membered heterocyclic ring having no additional heteroatom or one additional oxygen or nitrogen atom; a radical -OY₃, wherein Y₃ is hydrogen; or C₁-C₁₂-alkyl which is unsubstituted or substituted by -NR₂₃R₂₃'; or is a radical -C(O)-C₁-C₄-alkyl; and wherein R₂₃ and R₂₃' are as defined above; or a five- to seven-membered heterocyclic radical having at least one N-atom and being bound in each case via said nitrogen atom.

Suitable anionic substituents of B or B' include for example C₁-C₆-alkyl which is substituted by -SO₃H, -OSO₃H, -OPO₃H₂ and -COOH; phenyl which is substituted by one or more same or different substituents selected from the group consisting of -SO₃H, -COOH, -OH and - CH₂-SO₃H; -COOH; a radical -COOY₄, wherein Y₄ is C₁-C₂₄-alkyl which is substituted for example by -COOH, -SO₃H, -OSO₃H, -OPO₃H₂ or by a radical -NH-C(O)-O-G' wherein G' is the radical of an anionic carbohydrate; a radical -CONY₅Y₆ wherein Y₅ is C₁-C₂₄-alkyl which is substituted by -COOH, -SO₃H, -OSO₃H, or -OPO₃H₂ and Y₆ independently has the meaning of Y₅ or is hydrogen or C₁-C₁₂-alkyl; or -SO₃H; or a salt thereof, for example a sodium, potassium, ammonium or the like salt thereof.

Suitable cationic substituents of B or B' include C₁-C₁₂-alkyl which is substituted by a radical - NR₂₃R₂₃'R₂₃"⁺An⁻, wherein R₂₃, R₂₃' and R₂₃" are each independently of another hydrogen or unsubstituted or hydroxy-substituted C₁-C₆-alkyl or phenyl, and An⁻ is an anion; or a radical - C(O)OY₇, wherein Y₇ is C₁-C₂₄-alkyl which is substituted by -NR₂₃R₂₃'R₂₃"⁺An⁻ and is further unsubstituted or substituted for example by hydroxy, wherein R₂₃ R₂₃', R₂₃'' and An⁻ are as defined above.

Suitable zwitterionic substituents of B or B' include a radical -R₂₄-Zw, wherein R₂₄ is a direct bond or a functional group, for example a carbonyl, carbonate, amide, ester, dicarboanhydride, dicarboimide, urea or urethane group; and Zw is an aliphatic moiety comprising one anionic and one cationic group each.

The following preferences apply to the hydrophilic substituents of B and B':

### (i) non-ionic substituents:

Preferred alkyl substituents of B or B' are C₁-C₄-alkyl, in particular C₁-C₂-alkyl, which is substituted by one or more substituents selected from the group consisting of -OH and - NR₂₃R₂₃', wherein R₂₃ and R₂₃' are each independently of another hydrogen or C₁-C₄-alkyl, preferably hydrogen, methyl or ethyl and particularly preferably hydrogen or methyl, for example -CH₂-NH₂, -CH₂-N(CH₃)₂.
Preferred phenyl substituents of B or B' are phenyl which is substituted by -NH₂ or N(C₁-C₂-alkyl)₂, for example o-, m- or p-aminophenyl.
In case that the hydrophilic substituent of B or B' is a radical -COOY, Y as optionally substituted alkyl is preferably C₁-C₁₂-alkyl, more preferably C₁-C₆-alkyl, even more preferably C₁-C₄-alkyl and particularly preferably C₁-C₂-alkyl, each of which being unsubstituted or substituted as mentioned above. In case that the alkyl radical Y is substituted by -NR₂₃R₂₃', the above-given meanings and preferences apply for R₂₃ and R₂₃'. Examples of suitable saccharide substituents -O-G of the alkyl radical Y that is substituted by -NH-C(O)-O-G are the radical of a mono- or disaccharide, for example glucose, acetyl glucose, methyl glucose, glucosamine, N-acetyl glucosamine, glucono lactone, mannose, galactose, galactosamine, N-acetyl galactosamine, fructose, maltose, lactose, fucose, saccharose or trehalose, the radical of an anhydrosaccharide such as levoglucosan, the radical of a glucosid such as octylglucosid, the radical of a sugar alcohol such as sorbitol, the radical of a sugar acid derivative such as lactobionic acid amide, or the radical of an oligosaccharide with a maximum of 8 sugar units, for example fragments of a cyclodextrin, starch, chitosan, maltotriose or maltohexaose. The radical -O-G preferably denotes the radical of a mono- or disaccharide or the radical of a cyclodextrin fragment with a maximum of 8 sugar units. Particular preferred saccharide radicals -O-G are the radical of trehalose or the radical of a cyclodextrin fragment. In case that the alkyl radical Y is substituted by a radical -O-(CH₂CH₂O)₁₋₂₄-E or -NH-C(O)-O-G wherein -O-G is -O-(CH₂CH₂O)₁₋₂₄-E, the number of (CH₂CH₂O) units is preferably from 1 to 12 in each case and more preferably from 2 to 8. E is preferably hydrogen or C₁-C₂-alkyl.
Y as C₅-C₈-cycloalkyl is for example cyclopentyl or preferably cyclohexyl, each of which being unsubstituted or substituted for example by 1 to 3 C₁-C₂-alkyl groups.Y as C₇-C₁₂-aralkyl is for example benzyl.

Preferred nonionic radicals -COOY are those wherein Y is C₁-C₆-alkyl; or C₂-C₆-alkyl which is substituted by one or two substituents selected from the group consisting of hydroxy; ; C₁-C₂-alkoxy; -O-Si(CH₃)₃; and -NR₂₃R₂₃' wherein R₂₃ and R₂₃' are each independently of another hydrogen or C₁-C₄-alkyl; or Y is a radical -CH₂CH₂-O-(CH₂CH₂O)₁₋₁₂-E wherein E is hydrogen or C₁-C₂-alkyl; or is a radical -C₂-C₄-alkylene-NH-C(O)-O-G, wherein -O-G is the radical of a saccharide.

More preferred non-ionic radicals -COOY are those wherein Y is C₁-C₄-alkyl; or C₂-C₄-alkyl which is substituted by one or two substituents selected from the group consisting of -OH and -NR₂₃R₂₃' wherein R₂₃ and R₂₃' are each independently of another hydrogen or C₁-C₂-alkyl; or a radical -CH₂CH₂-O-(CH₂CH₂O)₁₋₁₂-E wherein E is hydrogen or C₁-C₂-alkyl; or is a radical -C₂-C₄-alkylene-NH-C(O)-O-G wherein -O-G is the radical of a saccharide.

Particularly preferred radicals -COOY comprise those wherein Y is C₁-C₂-alkyl, particularly methyl; or C₂-C₃-alkyl, which is unsubstituted or substituted by hydroxy or N,N-di-C₁-C₂-alkylamino, or is a radical -C₂-C₃-alkylene-NH-C(O)-O-G wherein -O-G is the radical of trehalose or the radical of a cyclodextrin fragment with a maximum of 8 sugar units.

Preferred non-ionic substituents -C(O)-NY₁Y₂ of B or B' are those wherein Y₁ and Y₂ are each independently of the other hydrogen or C₁-C₆-alkyl which is unsubstituted or substituted by hydroxy; or Y₁ and Y₂ together with the adjacent N-atom form a heterocyclic 6-membered ring having no further heteroatom or having one further N- or O-atom. Even more preferred meanings of Y₁ and Y₂, independently of each other, are hydrogen or C₁-C₄-alkyl which is unsubstituted or substituted by hydroxy; or Y₁ and Y₂ together with the adjacent N-atom form a N-C₁-C₂-alkylpiperazino or morpholino ring. Particularly preferred non-ionic radicals -C(O)-NY₁Y₂ are those wherein Y₁ and Y₂ are each independently of the other hydrogen or C₁-C₂-alkyl; or Y₁ and Y₂ together with the adjacent N-atom form a morpholino ring.

Preferred non-ionic substituents -OY₃ of B or B' are those wherein Y₃ is hydrogen, C₁-C₄-alkyl which is unsubstituted or substituted by -NH₂ or -N(C₁-C₂-alkyl)₂, or is a group - C(O)C₁-C₂-alkyl. Y₃ is particularly preferred hydrogen or acetyl.

Preferred non-ionic heterocyclic substituents of B or B' are a 5- or 6-membered heteroaromatic or heteroaliphatic radical having one N-atom and in addition no further heteroatom or an additional N- or O- heteroatom, or is a 5 to 7-membered lactame. Examples of such heterocyclic radicals are N-pyrrolidonyl, 2- or 4-pyridinyl, 2-methyl pyridin-5-yl, 2-, 3- oder 4-hydroxypyridinyl, N-ε-caprolactamyl, N-imidazolyl, 2-methylimidazol-1-yl, N-morpholinyl or 4-N-methylpiperazin-1-yl, particularly N-morpholinyl or N-pyrrolidonyl.

A group of preferred non-ionic substituents of B or B' comprises C₁-C₂-alkyl, which is unsubstituted or substituted by -OH or -NR₂₃R₂₃', wherein R₂₃ and R₂₃' are each independently of the other hydrogen or C₁-C₂-alkyl; a radical -COOY wherein Y is C₁-C₄-alkyl; C₂-C₄-alkyl which is substituted by -OH, -NR₂₃R₂₃' wherein R₂₃ and R₂₃' are each independently of another hydrogen or C₁-C₂-alkyl, or Y is a radical -C₂-C₄-alkylene-NH-C(O)-O-G wherein -O-G is the radical of a saccharide; a radical -C(O)-NY₁Y₂, wherein Y₁ and Y₂ are each independently of the other hydrogen or C₁-C₆-alkyl which is unsubstituted or substituted by hydroxy, or Y₁ and Y₂ together with the adjacent N-atom form a heterocyclic 6-membered ring having no further heteroatom or having one further N- or O-atom; a radical - OY₃, wherein Y₃ is hydrogen, C₁-C₄-alkyl which is unsubstituted or substituted by -NH₂ or - N(C₁-C₂-alkyl)₂, or is a group -C(O)C₁-C₂-alkyl; or a 5- or 6-membered heteroaromatic or heteroaliphatic radical having one N-atom and in addition no further heteroatom or an additional N-, O- or S-heteroatom, or a 5 to 7-membered lactame.

A group of more preferred non-ionic substituents of B or B' comprises a radical -COOY, wherein Y is C₁-C₂-alkyl, C₂-C₃-alkyl, which is substituted by hydroxy, amino or N,N-di-C₁-C₂-alkylamino, or is a radical -C₂-C₄-alkylene-NH-C(O)-O-G wherein -O-G is the radical of trehalose; a radical -CO-NY₁Y₂, wherein Y₁ and Y₂ are each independently of the other hydrogen or C₁-C₄-alkyl which is unsubstituted or substituted by hydroxy, or Y₁ and Y₂ together with the adjacent N-atom form a N-C₁-C₂-alkylpiperazino or morpholino ring; or a heterocyclic radical selected from the group consisting of N-pyrrolidonyl, 2- or 4-pyridinyl, 2-methylpyridin-5-yl, 2-, 3- oder 4-hydroxypyridinyl, N-ε-caprolactamyl, N-imidazolyl, 2-methylimidazol-1-yl, N-morpholinyl and 4-N-methylpiperazin-1-yl.

A particularly preferred group of non-ionic substituents of B or B' comprises the radicals - CONH₂, -CON(CH₃)₂, 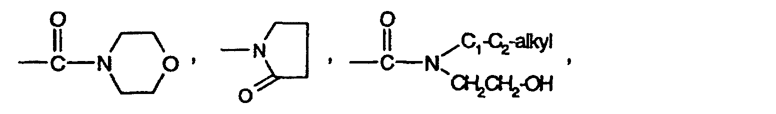 - CONH-(CH₂)₂-OH, -COO-(CH₂)₂-N(CH₃)₂, and -COO(CH₂)₂₋₄-NHC(O)-O-G wherein -O-G is the radical of trehalose.

### (ii) anionic substituents:

Preferred anionic substituents of B or B' are C₁-C₄-alkyl, in particular C₁-C₂-alkyl, which is substituted by one or more substituents selected from the group consisting of -SO₃H and - OPO₃H₂, for example -CH₂-SO₃H; phenyl which is substituted by -SO₃H or sulfomethyl, for example o-, m- or p-sulfophenyl or o-, m- or p-sulfomethylphenyl; -COOH; a radical - COOY₄, wherein Y₄ is C₂-C₆-alkyl which is substituted by -COOH, -SO₃H, -OSO₃H, - OPO₃H₂, or by a radical -NH-C(O)-O-G' wherein G' is the radical of lactobionic acid, hyaluronic acid or sialic acid, in particular C₂-C₄-alkyl which is substituted by -SO₃H or - OSO₃H; a radical -CONY₅Y₆ wherein Y₅ is C₁-C₆-alkyl substituted by sulfo, in particular C₂-C₄-alkyl substituted by sulfo, and Y₆ is hydrogen, for example the radical -C(O)-NH-C(CH₃)₂-CH₂-SO₃H; or -SO₃H; or a suitable salt thereof. Particular preferred anionic substituents of B or B' are -COOH, -SO₃H, o-, m- or p-sulfophenyl, o-, m- or p-sulfomethylphenyl or a radical - CONY₅Y₆ wherein Y₅ is C₂-C₄-alkyl substituted by sulfo, and Y₆ is hydrogen.

### (iii) cationic substituents:

Preferred cationic substituents of B or B' are C₁-C₄-alkyl, in particular C₁-C₂-alkyl, which is in each case substituted by -NR₂₃R₂₃'R₂₃"⁺An⁻; or a radical -C(O)OY₇ wherein Y₇ is C₂-C₆-alkyl, in particular C₂-C₄-alkyl, which is in each case substituted by -NR₂₃R₂₃'R₂₃"⁺An⁻ and is further unsubstituted or substituted by hydroxy. R₂₃, R₂₃' and R₂₃* are each independently of another preferably hydrogen or C₁-C₄-alkyl, more preferably methyl or ethyl and particularly preferably methyl. Examples of suitable anions An⁻ are Hal⁻, wherein Hal is halogen, for example Br⁻, F⁻, J⁻ or particularly Cl⁻, furthermore HCO₃⁻, CO₃²⁻, H₂PO₃⁻, HPO₃²⁻, PO₃³⁻, HSO₄⁻ , SO₄²⁻ or the radical of an organic acid such as OCOCH₃⁻ and the like. A particularly preferred cationic substituent of B or B' is a radical -C(O)OY₇ wherein Y₇ is C₂-C₄-alkyl, which is substituted by -N(C₁-C₂-alkyl)₃⁺An⁻ and is further substituted by hydroxy, and An⁻ is an anion, for example the radical -C(O)O-CH₂-CH(OH)-CH₂-N(CH₃)₃⁺An⁻.

### (iv) zwitterionic substituents -R₂₄-Zw:

R₂₄ is a preferably a carbonyl, ester or amide functional group and more preferably an ester group -C(O)-O-.
Suitable anionic groups of the moiety Zw are for example -COO⁻, -SO₃⁻, -OSO₃⁻, -OPO₃H⁻ or bivalent -O-PO₂⁻- or -O-PO₂⁻-O-, preferably a group -COO⁻ or -SO₃⁻ or a bivalent group - O-PO₂⁻-, and in particular a group -SO₃⁻.
Suitable cationic groups of the moiety Zw are for example a group -NR₂₃R₂₃'R₂₃"⁺ or a bivalent group -NR₂₃R₂₃'⁺-, wherein R₂₃, R₂₃' and R₂₃" are as defined above, and are each independently of the other, preferably hydrogen or C₁-C₆-alkyl, preferably hydrogen or C₁-C₄-alkyl and most preferably each methyl or ethyl.

The moiety Zw is for example C₂-C₃₀-alkyl, preferably C₂-C₁₂-alkyl, and more preferably C₃-C₈-alkyl, which is in each case uninterrupted or interrupted by -O- and substituted or interrupted by one of the above-mentioned anionic and cationic groups each, and, in addition, is further unsubstituted or substituted by a radical -OY₈, wherein Y₈ is hydrogen or the acyl radical of a carboxylic acid.

Y₈ is preferably hydrogen or the acyl radical of a higher fatty acid.

Zw is preferably C₂-C₁₂-alkyl and even more preferably C₃-C₈-alkyl which is substituted or interrupted by one of the above-mentioned anionic and cationic groups each, and in addition may be further substituted by a radical -OY₈.

A preferred group of zwitter-ionic substituents -R₂₄-Zw corresponds to the formula

-C(O)O-(alk''')-N(R₂₃)₂⁺-(alk')-An⁻

or

- C(O)O-(alk")-O-PO₂⁻-(O)₀₋₁-(alk'''')-N(R₂₃)₃⁺

wherein R₂₃ is hydrogen or C₁-C₆-alkyl; An⁻ is an anionic group -COO⁻, -SO₃⁻, -OSO₃⁻ or - OPO₃H⁻, preferably -COO⁻ or -SO₃⁻ and most preferably -SO₃⁻, alk' is C₁-C₁₂-alkylene, (alk") is C₂-C₂₄-alkylene which is unsubstituted or substituted by a radical -OY₈, Y₈ is hydrogen or the acyl radical of a carboxylic acid, and (alk''') is C₂-C₈-alkylene.

(alk') is preferably C₂-C₈-alkylene, more preferably C₂-C₆-alkylene and most preferably C₂-C₄-alkylene. (alk") is preferably C₂-C₁₂-alkylene, more preferably C₂-C₆-alkylene and particularly preferably C₂-C₃-alkylene which is in each case unsubstituted or substituted by hydroxy or by a radical -OY₈. (alk''') is preferably C₂-C₄-alkylene and more preferably C₂-C₃-alkylene. R₂₃ is hydrogen or C₁-C₄-alkyl, more preferably methyl or ethyl and particularly preferably methyl. A preferred zwitterionic substituent of B or B' is of formula

- C(O)O-CH₂-CH(OY₈)-CH₂-O-PO₂⁻-(CH₂)₂-N(CH₃)₃⁺,

wherein Y₈ is hydrogen or the acyl radical of a higher fatty acid.

B denotes for example a radical of formula 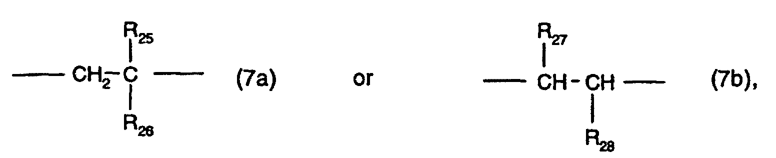 wherein R₂₅ is hydrogen or C₁-C₄-alkyl, preferably hydrogen or methyl; R₂₆ is a hydrophilic substituent, wherein the above given meanings and preferences apply; R₂₇ is C₁-C₄-alkyl, phenyl or a radical -C(O)OY₉, wherein Y₉ is hydrogen or unsubstituted or hydroxy-substituted C₁-C₄-alkyl; and R₂₈ is a radical -C(O)Y₉' or -CH₂-C(O)OY₉' wherein Y₉' independently has the meaning of Y₉.

R₂₇ is preferably C₁-C₂-alkyl, phenyl or a group -C(O)OY₉. R₂₈ is preferably a group - C(O)OY₉' or -CH₂-C(O)OY₉' wherein Y₉ and Y₉' are each independently of the other hydrogen, C₁-C₂-alkyl or hydroxy-C₁-C₂-alkyl. Particularly preferred -CHR₂₇-CHR₂₈- units according to the invention are those wherein R₂₇ is methyl or a group -C(O)OY₉ and R₂₈ is a
group -C(O)OY₉' or -CH₂-C(O)OY₉' wherein Y₉ and Y₉' are each hydrogen, C₁-C₂-alkyl or hydroxy-C₁-C₂-alkyl.

B' independently may have one of the meanings given above for B.

If (oligomer) is a radical of formula (6a), the radical -(alk)-S-[B]ₚ-[B']_{q}-Q preferably denotes a radical of formula 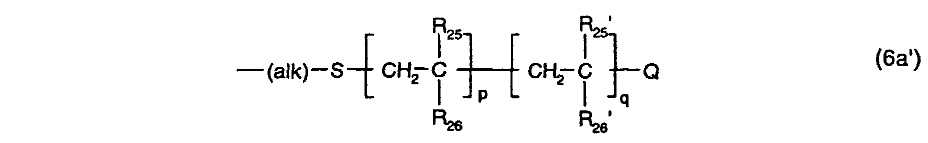 and even more preferably of the formula 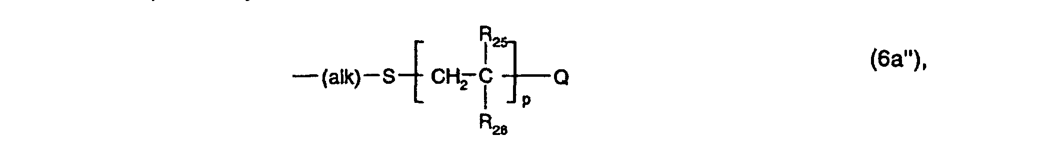 wherein for R₂₅, R₂₆, Q, p and q the above-given meanings and preferences apply, for R₂₅' independently the meanings and preferences given before for R₂₅ apply, and for R₂₆' independently the meanings and preferences given before for R₂₆ apply.

A preferred group of suitable hydrophilic macromonomers according to step (c) of the invention comprises compounds of formula 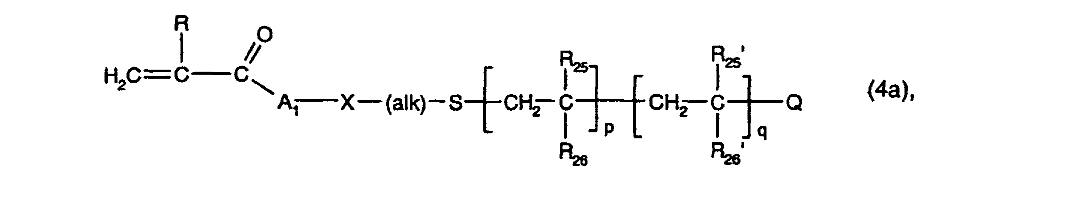 wherein R is hydrogen or methyl, A₁ is -O-(CH₂)₂₋₄-, -O-CH₂-CH(OH)-CH₂- or a radical -O-(CH₂)₂₋₄-NH-C(O)-, X is -O- or -NH-, (alk) is C₂-C₄-alkylene, Q is a monovalent group that is suitable to act as a polymerization chain-reaction terminator, p is an integer from 5 to 50, R₂₅ and R₂₅' are each independently of the other hydrogen or methyl, and for R₂₆ and R₂₆' each independently the above given meanings and preferences apply.

A particularly preferred embodiment of the invention relates to hydrophilic macromonomers of the formula 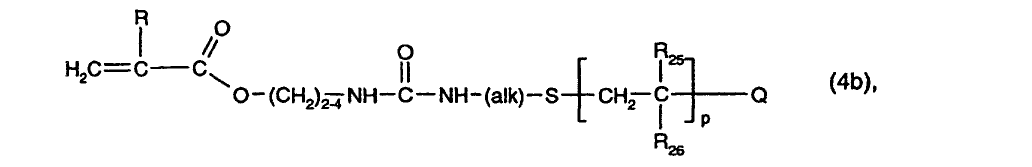 wherein for R, R₂₅, R₂₆, Q, (alk) and p the above-given meanings and preferences apply. A particularly preferred group of hydrophilic macromonomers are compounds of the above formula (4b) wherein R is hydrogen or methyl, (alk) is C₂-C₄-alkylene, R₂₅ is hydrogen or methyl, p is an integer of 5 to 50, Q is as defined before, and for R₂₆ the above given meanings and preferences apply; in particular R₂₆ of this embodiment is a radical -CONH₂, -CON(CH₃)₂ or

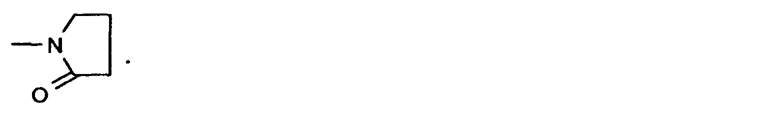

If (oligomer) is a radical (ii) of formula (6b), Q' in formula (6b) is for example C₁-C₁₂-alkyl, phenyl or benzyl, preferably C₁-C₂-alkyl or benzyl and in particular methyl. R₁₉ is preferably unsubstituted or hydroxy-substituted C₁-C₄-alkyl and in particular methyl. u is preferably an integer from 2 to 150, more preferably from 5 to 100, even more preferably from 5 to 75 and particularly preferably from 10 to 50.

If (oligomer) is a radical of formula (6b'), the above given meanings and preferences apply for the variables R₁₉ and u contained therein. X in formula (6b') is preferably hydroxy or amino.

If (oligomer) denotes a radical (iv) of formula (6c), R₂₀ and R₂₀' are each preferably ethyl or in particular methyl; v is preferably an integer from 2 to 150, more preferably from 5 to 100, even more preferably from 5 to 75 and particularly preferably from 10 to 50; Q" is for example hydrogen; and An⁻ is as defined before.

If (oligomer) denotes an oligopeptide radical (v) of formula (6d) or 6d'), R₂₁ is for example hydrogen, methyl, hydroxymethyl, carboxymethyl, 1-hydroxyethyl, 2-carboxyethyl, isopropyl, n-, sec. or iso-butyl, 4-amino-n-butyl, benzyl, p-hydroxybenzyl, imidazolylmethyl, indolylmethyl or a radical -(CH₂)₃-NH-C(=NH)-NH₂. t is preferably an integer from 2 to 150, more preferably from 5 to 100, even more preferably from 5 to 75 and particularly preferably from 10 to 50.

If (oligomer) denotes a polyoxyalkylene radical (vi) of formula (6e), R₃₄ is preferably hydrogen or C₁-C₁₈-alkyl, more preferably hydrogen or C₁-C₁₂-alkyl, even more preferably hydrogen, methyl or ethyl, and particularly preferably hydrogen or methyl. (alk") is preferably a C₂-C₃-alkylene radical. z is preferably 0. r and s are each independently preferably an integer from 0 to 100 wherein the total of (r+s) is 5 to 100. r and s are each independently more preferably an integer from 0 to 50 wherein the total of (r+s) is 8 to 50. In a particularly preferred embodiment of the polyoxyalkylene radicals (oligomer), r is an integer from 8 to 50 and particularly 9 to 25, and s is 0.

(oligomer) as the radical of an oligosaccharide (vii) may be, for example, a di- or polysaccharide including carbohydrate containing fragments from a biopolymer. Examples are the radical of a cyclodextrin, trehalose, cellobiose, maltotriose, maltohexaose, chitohexaose or a starch, hyaluronic acid, deacetylated hyaluronic acid, chitosan, agarose, chitin 50, amylose, glucan, heparin, xylan, pectin, galactan, glycosaminoglycan, mucin, dextran, aminated dextran, cellulose, hydroxyalkylcellulose or carboxyalkylcellulose oligomer, each of which with a molecular weight average weight of, for example, up to 25000, preferably up to 10000. Preferably the oligosaccharide according to (vii) is the radical of a cyclodextrin with a maximum of 8 sugar units.

Formulae (6a), (6a') or (6e) are to be understood as a statistic description of the respective oligomeric radicals, that is to say, the orientation of the monomers and the sequence of the monomers (in case of copolymers) are not fixed in any way by said formulae. The arrangement of B and B' in formula (6a) or of the ethyleneoxide and propyleneoxide units in formula (6e) thus in each case may be random or blockwise.

The weight average molecular weight of the hydrophilic macromonomer according to step (c) depends principally on the desired properties and is for example from 300 to 25000, preferably from 300 to 12000, more preferably from 300 to 8000, even more preferably from 300 to 5000, and particularly preferably from 500 to 4000.

The macromonomers of formula (4) may be prepared by methods known per se. For example, the compounds of formula (4) wherein A is a radical of formula (5a), (5b) or (5d) are obtainable by reacting a compound of formula 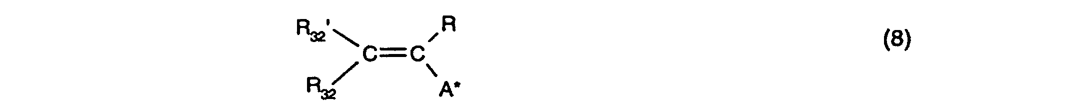 wherein R, R₃₂ and R₃₂' each have the above-given meaning and A* is, for example, a group -C(O)-A**, wherein A** is halogen, particularty chlorine, an ester group an oxyalkylene radical comprising an epoxy group, for example the radical 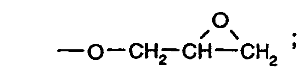 or is a radical -O-C₂-C₁₂-alkylene-N=C=O; or A* is a radical - (A₂)ₘ-N=C=O, wherein A₂ and m have the above-given meaning, with a compound of formula

HX - (oligomer) (9),

wherein X has the above-given meaning.

The reactions of a compound of formula (8) having a carboxylic acid halide group, an epoxy group or an isocyanato group with an amino or hydroxy compound of formula (9) are well-known in the art and may be carried out as desribed in textbooks of organic chemistry. For example, the reaction of an isocyanato derivative of formula (8) with a compound of formula (9) may be carried out in an inert organic solvent such as an optionally halogenated hydrocarbon, for example petroleum ether, methylcyclohexane, toluene, chloroform, methylene chloride and the like, or an ether, for example diethyl ether, tetrahydrofurane, dioxane, or a more polar solvent such as DMSO, DMA, N-methylpyrrolidone or even a lower alcohol, at a temperature of from 0 to 100°C, preferably from 0 to 50°C and particularly preferably at room temperature, optionally in the presence of a catalyst, for example a tertiary amine such as triethylamine or tri-n-butylamine, 1,4-diazabicyclooctane, or a tin compound such as dibutyltin dilaurate or tin dioctanoate. In addition, the reaction of an isocyanato derivative of formula (8) with a compound of formula (9) wherein -XH is an amino group also may be carried out in an aqueous solution in the absence of a catalyst. It is advantageous to carry out the above reactions under an inert atmosphere, for example under an nitrogen or argon atmosphere.

Moreover, the macromonomers of formula (4) wherein A is a radical of formula (5c) or (5e) may be obtained by reacting a compound of formula 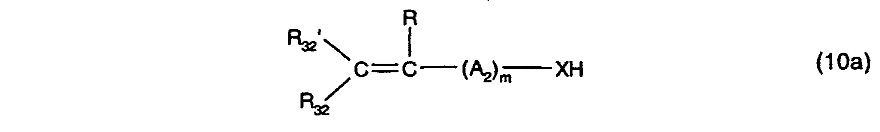 or 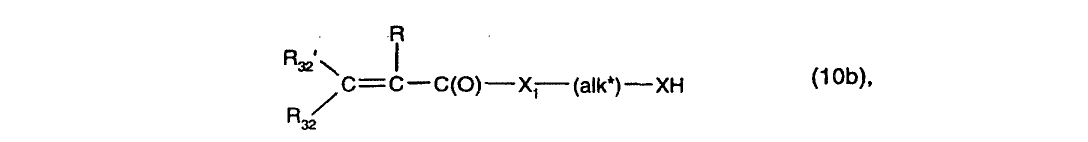 wherein R, R₃₂, R₃₂', A₂, X, X₁, (alk*) and m each have the above-given meaning, with a compound of formula

- X₁'(O)C - (oligomer) (9a),

wherein (oligomer) has the above-given meaning and X₁' is for example -OH or halogen, in particular chlorine, or together with -(O)C- forms an anhydride group, in a manner known per se.

The macromonomers of formula (4), wherein A is a direct bond and (oligomer) is a radical of formula (6c') are known or may be prepared according to methods known in the art, for example as described in S. Kobayashi et al., Polymer Bulletin 13, p 447-451 (1985).

Likewise, the macromonomers of the formula 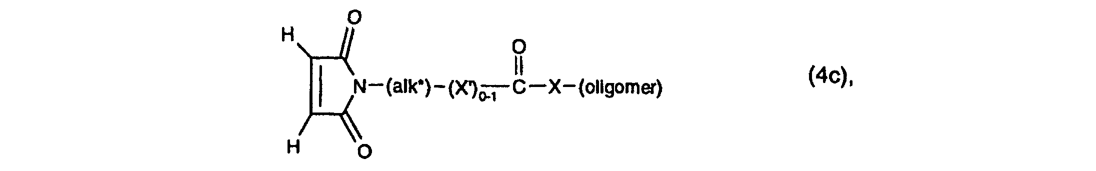 wherein (alk*), X', X and (oligomer) each have the above-given meaning, may be obtained in a manner known per se, for example, by reacting a compound of formula 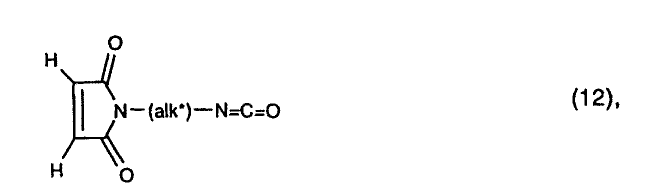 wherein (alk*) has the above-given meaning, with a compound of the above-given formula (6), or by reacting a compound of formula 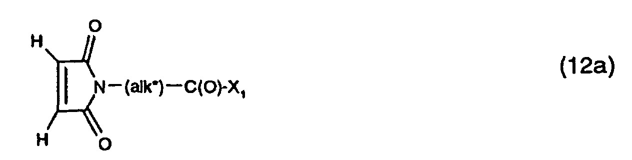 with a compound of the above formula (9) wherein (alk*) and X₁ each have the above-given meaning.

The compounds of the formula (8), (9), (9a), (10a), (10b), (12) and (12a) are known compounds which are commercially available or may be prepared according to known methods. For example, compounds of the formula (9) and (9a) wherein (oligomer) denotes a radical of formula (6a) may be prepared according to PCT application WO 92/09639 by copolymerizing one or more hydrophilic ethylenically unsaturated monomers in the presence of a functional chain transfer agent such as cysteamine hydrochloride, thioglycolic acid or the like.

The hydrophilic monomers or macromonomers may be applied to the initiator-modified material and polymerized there according to processes known per se. For example, the material comprising the covalently bound polymerisation initiator is immersed in a solution of the monomer or macromonomer, or a layer of monomer or macromonomer is first of all deposited on the modified material surface, for example, by dipping, spraying, spreading, knife coating, pouring, rolling, spin coating or vacuum vapor deposition. Suitable solvents, if used in the polymerization process, are, for example, water or dipolar aprotic solvents such as, for example, acetonitrile. The polymerization of the hydrophilic monomer or macromonomer on the material comprising the primary polymer coating then may be initiated , for example, thermally by the action of heat or preferably by irradiation, particularly by UV radiation. Suitable light sources for the irradiation are known to the artisan and comprise for example mercury lamps, high pressure mercury lamps, xenon lamps, carbon arc lamps or sunlight. The time period of irradiation may depend for example on the desired properties of the resulting composite material but is usually in the range of up to 30 minutes, preferably from 10 secondes to 10 minutes, and particularly preferably from 0.5 to 5 minutes. It is advantageous to carry out the irradiation in an atmosphere of inert gas. After the polymerization, any non-covalently bound monomers, polymers, oligomers or non-reacted macromonomers formed can be removed, for example by treatment with suitable solvents.

The coated material obtained according to the invention may be purified afterwards in a manner known per se, for example by washing or extraction with a suitable solvent such as water.

By means of process step (c) of the above-described coating process, the hydrophilic macromonomers may be grafted to the material surface with formation of a coating having, for example, a so-called bottle brush-type structure (BBT) composed of tethered "hairy" chains. Such BBT structures in one embodiment comprise a long hydrophilic or hydrophobic backbone which carries relatively densely packed comparatively short hydrophilic side chains (called primary bottle brushes). Another embodiment relates to secondary bottle brushes which are characterized in that the hydrophilic side chains themselves carry densely packed hydrophilic "secondary" side chains. Polymeric coatings of said primary and secondary BBT structures to a certain extent mimic highly water-retaining structures occurring in the human body, for example in cartilage or mucosal tissue.

The coating thickness of the macromonomers depends principally on the desired properties. It can be, for example, from 0.001 to 1000 µm, preferably from 0.005 to 100 µm, more preferably from 0.01 to 50 µm, even more preferably from 0.01 to 5 µm, especially preferably from 0.01 to 1 µm and particularly preferably from 0.01 to 0.5 µm.

A further embodiment of the invention relates to a material that is coated by the process of the invention.

The material that is coated by the process of the invention is, for example, an organic bulk material, preferably a biomedical device, e.g. an ophthalmic device, preferably a contact lens including both hard and particularly soft contact lenses, an intraocular lens or artificial cornea. Further examples are materials useful for example as wound healing dressings, eye bandages, materials for the sustained release of an active compound such as a drug delivery patch, moldings that can be used in surgery, such as heart valves, vascular grafts, catheters, artificial organs, encapsulated biologic implants, e.g. pancreatic islets, materials for prostheses such as bone substitutes, or moldings for diagnostics, membranes or biomedical instruments or apparatus.

The biomedical devices, e.g. ophthalmic devices obtained according to the invention have a variety of unexpected advantages over those of the prior art which make those devices very suitable for practical purposes,e.g. as contact lens for extended wear or intraocular lens. For example, they do have a high surface wettability which can be demonstrated by their contact angles, their water retention and their water-film break up time or tear film break up time (TBUT).

The TBUT plays an particularly important role in the field of ophthalmic devices such as contact lenses. Thus the facile movement of an eyelid over a contact lens has proven important for the comfort of the wearer; this sliding motion is fa cilitated by the presence of a continuous layer of tear fluid on the contact lens, a layer which lubricates the tissue/lens interface. However, clinical tests have shown that currently available contact lenses partially dry out between blinks, thus increasing friction between eyelid and the lens. The increased friction results in soreness of the eyes and reduced movement of the contact lenses. Now it has become feasible to considerably increase the TBUT of commercial contact lenses such as, for example, those made of nelfilcon A, vifilcon A or lotrafilcon A polymer, by applying a surface coating according to the invention. On the base curve of a contact lens, the pronounced lubricity of the coating facilitates the on-eye lens movement which is essential for extended wear of contact lenses. Moreover, the materials obtained by the process of the invention provide additional effects being essential for lenses for extended wear, such as an increased thickness of the pre-lens tear film which contributes substantially to low microbial adhesion and resistance to deposit formation. Due to the extremely soft and lubricious character of the novel surface coatings, biomedical articles such as in particular contact lenses coated by the process of the invention show a superior wearing comfort including improvements with respect to late day dryness and long term (overnight) wear. The novel surface coatings moreover interact in a reversible manner with occular mucus which contributes to the improved wearing comfort.

In addition, biomedical devices, e.g. ophthalmic devices such as contact lenses, coated by the process of the invention, have a very pronounced biocompatibility combined with good mechanical properties. For example, the devices are blood compatible and have a good tissue integration. In addition, there are generally no adverse eye effects observed, while the adsorption of proteins or lipids is low, also the salt deposit formation is lower than with conventional contact lenses. Generally, there is low fouling, low microbial adhesion and low bioerosion while good mechanical properties can be for example found in a low friction coefficient and low abrasion properties. Moreover, the dimensional stability of the materials obtained according to the invention is excellent. In addition, the attachment of a hydrophilic surface coating at a given bulk material according to the invention does not affect its visual transparency.

In summary, the ophthalmic devices obtained by the process of the invention, such as contact lenses and artificial cornea, provide a combination of low spoilation with respect to cell debris, cosmetics, dust or dirt, solvent vapors or chemicals, with a high comfort for the patient wearing such opthalmic devices in view of the soft hydrogel surface which for example provides a very good on-eye movement of the ohthalmic device.

Biomedical devices such as renal dialysis membranes, blood storage bags, pacemaker leads or vascular grafts coated by the process of the invention resist fouling by proteins by virtue of the continuous layer of bound water, thus reducing the rate and extent of thrombosis. Blood-contacting devices fabricated according to the present invention are therefore haemocompatible and biocompatible.

In the examples, if not indicated otherwise, amounts are amounts by weight, temperatures are given in degrees Celsius. Tear break-up time values in general relate to the pre-lens tear film non-invasive break-up time (PLTF-NIBUT) that is determined following the procedure published by M. Guillon et al., Ophthal. Physiol. Opt. 9, 355-359 (1989) or M. Guillon et al., Optometry and Vision Science 74, 273-279 (1997). Average advancing and receding water contact angles of coated and non-coated lenses are determined with the dynamic Wilhelmy method using a Krüss K-12 instrument (Krüss GmbH, Hamburg, Germany). Wetting force on the solid is measured as the solid is immersed in or withdrawn from a liquid of known surface tension.

### Examples A1-A4: Spray coating on contact lenses using azido aniline hydrochloride

A solution of 0.1 mg/ml azido aniline hydrochloride in methanol is given into a funnel of an airbrush (aero-pro 381™, Hansa). The solution is sprayed onto both sides of wet or dried lotrafilcon A lenses (polysiloxane/perfluoroalkylpolyether copolymer) for the time as indicated in the Table below using a nitrogen pressure of 1.15 bar. Afterwards the lenses are irradiated 30 seconds using a UV lamp (LQ 400B, Gröbel) with an intensity of 1.43 mW/cm² and a 305 nm cutoff filter. The whole process is optionally repeated. The lenses are then extracted in acetonitrile/methanol 80/20 overnight.

**Table**

| Example | Spray time in seconds/ number of spray cycles | Lens surfaces before spraying |
|---|---|---|
| A-1 | 3/1 | dry |
| A-2 | 7/1 | dry |
| A-3 | 7/1 | wet |
| A-4 | 7/3 | dry |

### Example A-5: Surface Functionalization of contact lenses using a benzophenone

Uncoated lotrafilcon A silicone-hydrogel contact lenses are placed in a 3 cm Petri dish and treated with 10 ml of a 2 % w/w solution of benzophenone-3,4,3',4'-tetracarboxylic acid dianhydride (BTDA) in formamide by gentle shaking for 6 minutes. The Petri dish is then exposed to UV irradiation for 2 minutes under ambient conditions using a Groebel RM-3 lamp. Excessive BTDA is removed from the lens surfaces by repeated rinses with formamide and water.

### Example A-6: Surface Functionalization of contact lenses using a benzophenone

A drop of the BTDA solution as prepared in Example A-5 is placed in the female part of a polypropylene (PP) contact lens mold. A lotrafilcon A contact lens is then placed into that mold on a way that the BTDA solution forms a thin capillary layer between mold surface and lens surface. A second drop of the BTDA solution is placed in the cavity of the lens and the PP mold is finally closed by putting it's male part on top. The mold is only weakly clamped in order to maintain capillary layers of BTDA solution on both sides of the contact lens. The molds are then simultaneously UV irradiated from both sides for 60 seconds. After removal from the molds the contact lenses thus treated are rinsed with formamide and water and finally autoclaved in water to 30 minutes at 121 °C.

### Example A-7: Surface Funtionalization of contact lenses using a benzophenone

As described in Examples A-5 and A-6 lotrafilcon A contact lenses are treated with a BTDA solution in formamide which contains in addition 0,2 % of the surfactant Silwet L77 (Wacker, Burghausen/Germany). The lenses are dipped 3-times for 30 seconds in the solution, placed onto a polypropylene film, then UV irradiated for 2 minutes and rinsed.

### Example A-8: Surface Functionalization of contact lenses using a benzophenone

Lotrafilcon A contact lenses are sprayed on both sides with a 10 % w/w solution of benzophenone-tetracarboxylic acid sodium salt (BTA-Na) in water, using a commercially available paint brush. The lenses are then UV irradiated for 1 minute, rinsed 3-times in water and autoclaved in water at 121 °C for 30 minutes. The uniformity of the surface functionalization, the polarity of the lens surfaces as well as their overall functionality can be improved by applying repeated spray/UV-irradiation cycles to the lenses.

### Example A-9: Surface functionalization of contact lenses using a benzophenone

According to the method described in Example A-8 lotrafilcon A contact lenses were spray/UV- treated in repeated cycles using a 10 % w/w solution of BTDA in THF, methylethylketone (MEK) or dimethylacetamide (DMAc).

### Examples A-10 ― A-13: Quantification of BTDA surface groups on contact lenses by spin-labelling and ESR-Spectroscopy

Anhydride functionalized lenses are prepared as described in examples A-5 ― A-9 (without autoclaving) and then treated at 25 °C for 10 hours with a 1 % w/w solution of the spin label 4-amino-2,2,6,6-tetramethyl-piperidine-N-oxide (4-amino-TEMPO) in acetonitrile. After careful extraction of only physically adsorbed excessive spin label molecules the lenses are investigated by ESR-spectroscopy. The concentration of functional anhydride groups on the lens surfaces is extrapolated from the total number of mmoles of bound nitroxyl radicals per lens.

| Example No. | Funtionalized lenses from Example No. | Concentration of anhydride groups [anhydride groups / nm²] |
|---|---|---|
| A-10 | A-5 | 26,3 |
| A-11 | A-6 | 13,2 |
| A-12 | A-7 | 5,8 |
| A-13 | A-9 | 7,3 |

### Examples A-14 ― A-15: Surface functionalization of contact lenses using 3,3'-Diamino-benzophenone (3,3'-DAB) and 3,4-Diamino-benzophenone (3,4-DAB)

As outlined in Examples A-8 and A-9 lotrafilcon A contact lenses are functionalized by spray-treament/UV-irradiation with 5 % w/w aqueous solutions of the 3,3'-DAB hydrochloride (A-14) or 3,4-DAB hydrochloride (A-15) using in each case 4 repeated cycles of spraying and UV irradiation. After careful rinsing with water the lenses are treated at 25 °C for 30 minutes with a 10 % w/w solution of triethylamine in acetonitrile.

### Examples B1 ― B-4: Surface binding of reactive photoinitiator molecules

The aminofunctionalized contact lenses from Examples A-1 ― A-4 are immersed into a 1 % by weight solution of the reactive photoinitiator prepared by the addition reaction from isophorone diisocyanate and 4-(2-hydroxyethoxy)phenyl 2-hydroxy-2-propyl ketone (Darocure 2959) (synthesis see EP 0 632 329) in acetonitrile. 3 drops of triethylamine (TEA) are then added to the solution. The amino groups on the lens surface react with the isocyanato groups of the photoinitiator molecules for 12 hours. After this time, the lenses are withdrawn from the reaction solution, 3x washed and extracted in acetonitrile for 8 hours and dried under reduced pressure for 2 hours. The dried lenses are subsequently used for photografting.

### Example B-5 - B-8: Surface binding of the reactive photoinitiator molecules

The aminofunctionalized contact lenses from Examples A-1 to A-4 are dried to the constant mass under reduced pressure. The lenses are then directly immersed into 1% by weight acetonitrile solution of the reactive photoinitiator prepared by the addition reaction from isophorone diisocyanate and 2-dimethylamino-2-benzyl-1-[4-(2-hydroxyethoxy)phenyl]-butan-1-one (synthesis see WO 96/20796 (5 ml solution/lens). 3 drops of triethylamine (TEA) are then added to the solution. The amino groups on the lens surface react with the isocyanato groups of the photoinitiator molecules for 12 hours. After this time, the lenses are withdrawn from the reaction solution, 3x washed and extracted in acetonitrile for 6 hours and dried under reduced pressure for 2 hours. The dried lenses are subsequently used for photografting.

### Example B-9: Surface binding of the reactive photoinitiator molecules

Using the method outlined in Example B-1 surface funtionalized lotrafilcon A contact lenses prepared in Example A-15 are treated with a 1% w/w acetonitrile solution of the reactive photoinitiator. The dried lenses are subsequently used for photografting.

### Example C-1: Acrylamide telomer (Mₙ 2000 Da) synthesis

A 1000 ml round bottom flask is charged with a solution of 71.1g (1 mol) acrylamide, 4.93g (18.2 mmol) α,α'-azodiisobutyramidine dihydrochloride and 4.93 g (36.4 mmol) cysteaminhydrochloride in 400 ml of water. The clear and slightly yellowish solution is acidified with a few drops of hydrochloric acid to pH3. The stirred acidic solution is evacuated to 50 mbar and filled with argon. This is repeated three times. With a constant stream of Argon, this solution is poured into a 500 ml dropping funnel which is put onto an 'flow-through-reactor' consisting of an 1000ml three-necked round-bottom flask, reflux condenser, thermometer, magnetic stirrer and a 30 cm Liebig-condenser, which is filled with glass wool. The whole apparatus is constantly purged with argon. The dropping funnel is put onto the Liebig condenser, which is heated to 65°C. The flask is heated to 60°C. The solution is slowly dropped through the Liebig-condenser into the stirred flask. This takes 2.5 hrs. During this time the temperature in the flask is kept between 58-65°C. After the completed addition, the solution is stirred for 2hrs at 60°C.
NaOH is added to the clear and slightly yellowish solution until pH 10 is reached. The product is purified through reverse osmosis, using Millipore cartridge with a cut-off at 1000 Da and freeze-dried. A bright-white solid product is obtained (NH₂ 0.34mEq/g ,sulfur-value of the elemental analysis (0.33mEq/g); Mₙ 2000 Da).

### Example C-2: Acrylamide telomer (Mₙ 1350 Da) synthesis

A 1000 mL round bottom flask is charged with a solution of 99.5 g (1.46 mol) acrylamide, 1.27 g (4.68 mmol) α,α'-azodiisobutyramidine dihydrochloride and 15.9 g (0.14 mol) cysteaminhydrochloride in 300 ml of water. The clear and slightly yellowish solution is acidified with a few drops of hydrochloric acid (32%) to pH 3. The stirred acidic solution is evacuated to 50 mbar and filled with argon. This is repeated three times. With a constant stream of argon, this solution is poured into a 500 ml dropping funnel which is put onto an 'flow-through-reactor' consisting of an 1000ml three-necked round-bottom flask, reflux condenser, thermometer, magnetic stirrer and a 30 cm Liebig-condenser, which is filled with glass wool. The whole apparatus is constantly purged with argon. The dropping funnel is put onto the Liebig condenser, which is heated to 65°C. The flask is heated to 60°C. The solution is slowly dropped through the Liebig-condenser into the stirred flask. This takes 2 hrs. During this time the temperature in the flask is kept between 58-65°C. After the completed addition, the solution is stirred for 2 hrs at 60°C.
NaOH is added to the clear and slightly yellowish solution until pH 10 is reached. The product is purified through reverse osmosis, using Millipore cartridge with a cut-off at 1000 Da and then freeze-dried for 18 hrs. A bright-white solid product is obtained (NH₂ 0.70mEq/g, sulfur-value of the elemental analysis (0.73mEq/g; Mₙ 1350 Da).

### Example C-3: N,N-dimethylacrylamide telomer (Mn 1850) synthesis

A 2000 mL round bottom flask is charged with a solution of 198.2 g (2 mol) N,N-dimethylacrylamide (DMA, 2.72 g (10 mmol)) α,α'-azodiisobutyramidine dihydrochloride and 24.8 g (0.22 mol) cysteamine hydrochloride in 600 ml of water.
The clear and slightly yellowish solution is acidified with a few drops of hydrochloric acid to pH3. The stirred acidic solution is evacuated to 50 mbar and filled with argon. This is repeated three times.
With a constant stream of Argon, this solution is poured into a 1000 ml dropping funnel which is put onto an 'flow-through-reactor' consisting of an 1000ml three-necked round-bottom flask, reflux condenser, thermometer, magnetic stirrer and a 30 cm Liebig-condenser, which is filled with glass wool. The whole apparatus is constantly purged with Argon.
The dropping funnel is put onto the Liebig condenser, which is heated to 60°C. The flask is also heated to 60°C. The solution is slowly dropped through the Liebig-condenser into the stirred flask. This takes about 2.5 hrs. During this time the temperature in the flask is kept between 58-65°C. After the completed addition, the solution is stirred for 2hrs at 60°C. 30 % NaOH solution is added to the clear and slightly yellowish solution until pH 10 is reached. The product is purified through reverse osmosis, using Millipore cartridge with a cut-off at 1000 Da and freeze-dried. A bright-white solid product is obtained. The concentration of amino groups is determined via functional group titration (0.54mEq/g). Mₙ ∼1850 g/Mol.

### Example D-1: Preparation of IEM-functionalized acrylamide telomer solution

7.5 g of acrylamide telomer with amino end group (amine titration = 0.70 mEq/g), prepared by Example C-2 are dissolved in 80 ml of HPLC water. Argon is then let to bubble through the solution for the period of about 30 minutes. This mixture is then added to the equimolar amount (0.81 g) of isocyanatoethyl methacrylate (IEM, isocyanate titration = 6.45 mEq/g) under stirring. The whole mixture is then stirred under argon flow for 12 hours. After adding of 0.8 g of NaCl to the solution and 10 minutes stirring, the mixture is filtered through 0.45 µ m Teflon filter, degassed by repeated (3x) evacuation and bubbling with argon in order to remove oxygen and used for photografting.

### Example D-2 : Preparation of IEM-functionalized DMA telomer solution

15 g of DMA telomer with amino end group (amine titration = 0.54 mEq/g) from Example C-3 are dissolved in 100 ml of HPLC water. Argon is then let to bubble through the solution for the period of about 30 minutes. This mixture is then added to the equimolar amount (1.25 g) of IEM (isocyanate titration = 6.45 mEq/g) under stirring. The whole mixture is then stirred under argon flow for 12 hours. After adding of 1.0 g of NaCl to the solution and 10 minutes stirring, the mixture is filtered through 0.45 µm Teflon filter, degassed with nitrogen in order to remove oxygen and used for photografting.

### Examples E-1 - E-4: Photografting of IEM-functionalized acrylamide telomers onto a contact lens surface

1 ml of the IEM-functionalized acrylamide telomer solution from Example D-1 is introduced into small Petri dishes each of a volume of about 2 ml in a glove box. The dried lenses from Examples B-1 ― B-4, carrying covalently linked photoinitiator molecules on its surface, are then placed each into one such dish and an additional 0.5 ml of the degassed solution is added on the lens in order to cover the whole lens with the solution. After 10 minutes, the Petri dishes carrying a lens in the solution are exposed to 14.5 mW/cm² ultraviolet light for a period of about 1.5 minutes.
The modified lenses are then withdrawn from the solution, washed twice in destilled water, continuously extracted in ultra pure water for 16 h, autoclaved for 30 minutes ar 121°C and analyzed by AFM, ATR-FTIR and contact angle measurements.

| Lens from Example | Dynamic contact angle advancing/receding | Thickness (AFM) |
|---|---|---|
| B-1 | 30° / 0° | 40 nm |
| B-2 | 0° / 0° | 500 nm |
| B-3 | 0° / 0° | 300 nm |
| B-4 | 0° / 0° | 370 nm |

### Example E-5: Photografting of IEM-functionalized acrylamide telomers onto the contact lens surface under ambient conditions

In a laminar flow hood, 1 ml of the IEM-functionalized acrylamide telomer solution from Example D-1 is introduced into a small Petri dish of a volume of about 2 ml. The dried lens from Example B-1, carrying covalently linked photoinitiator molecules on its surface, is then placed into this solution and an additional 0.5 ml of the degassed solution is added on the lens in order to cover the whole lens with the solution. After 10 minutes, the Petri dish with the lens in the solution is exposed to 2.05 mW/cm² ultraviolet light (MACAM-UV-Lamp) for a period of 2.5 minutes. The modified lens is then withdrawn from the solution, washed twice in destilled water, continuously extracted in ultra pure water for 16 h and analyzed by AFM, ATR-FTIR and contact angle measurements.
The thickness of the coating is in the range of 350-400 nm as determined by AFM. Water/air contact angles on the modified lens are 0° adv., 0° rec., 0° hysteresis. In comparison, the contact angles of non-modified lens are 101° adv., 64° rec., 37° hysteresis. The lens holds a continuous water layer on the surface for over 1 minute.

### Example E-6 : Photografting of IEM-functionalized DMA telomers onto the lens surface

1 ml of the IEM-functionalized N,N-dimethylacrylamide telomer solution from Example D-2 is introduced into a small Petri dish of a volume of about 2 ml in a glove box. The dried lens from Example B-1, carrying covalently linked photoinitiator molecules on its surface, is then placed into this solution and an additional 0.5 ml of the degassed solution is added on the lens in order to cover the whole lens with the solution. After 10 minutes, the Petri dish with the lens in the solution is exposed to 14.5 mW/cm² ultraviolet light for a period of about 1.5 minutes. The lens is then turned over and the exposition is repeated by applying 14.5 mW/cm² UV light for an additional 1.5 minutes.
The modified lens is then withdrawn from the solution, washed twice in destilled water, continuously extracted in ultra pure water for 16 h and analyzed by AFM, ATR-FTIR and contact angle measurements.
The thickness of the coating is in the range of 400-450 nm as determined by AFM. Water/air contact angles on the modified lens are 14° adv., 9° rec., 5° hysteresis. In comparison, the contact angles of non-modified lens are 101° adv., 64° rec., 37° hysteresis.

### Example E-7: Photografting of IEM-functionalized acrylamide telomers onto the contact lens surface

The contact lenses of Example B-9 are photografted in an aqueous solution according to the method described in Example E-1 using the polyacrylamide macromonomer of Example D-1. Dynamic contact angles of the lenses are: advancing 0°/ receding 0°.

## Claims

1. A process for coating a material surface comprising the steps of:
(a) applying to the material surface a compound of formula 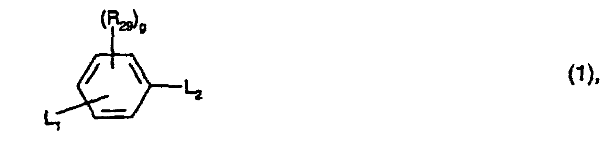 wherein R₂₉ is C₁-C₄-alkyl, C₁-C₄-alkoxy, hydroxy, sulfo, nitro, trifluoromethyl or halogen,
g is an integer from 0 to 2,
L₁ is a group, which functions as a triggerable precursor for carbene, nitrene or benzhydrol formation,
L₂ is amino, C₁-C₄-alkylamino, hydroxy, glycidyl, carboxy or a derivative thereof, isocyanato
or isothiocyanato, or is a radical of formula
- [L₃]ₕ - (spacer) - L₂' (1a),
or
L₂ and R₂₉ together form an anhydride radical 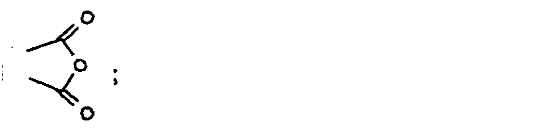 L₂' is amino, C₁-C₄-alkylamino, hydroxy, carboxy or a derivative thereof, isocyanato, isothiocyanato, -O-glycidyl or -O-C(O)-(CH₂)ₕ₁- X₂, wherein h1 is from 1 to 4 and X₂ is carboxy or a derivative thereof,
L₃ is -NH-, -NC₁-C₆-alkyl-, -O-, -C(O)O-, -C(O)NH-, -NHC(O)NH-, -NHC(O)O-or - OC(O)NH-;
(spacer) is linear or branched C₁-C₂₀₀-alkylene which may be substituted by hydroxy and/or interrupted by -O- except for C₁-alkyl, or is C₃-C₈-cycloalkylene, C₃-C₈-cycloalkylene-C₁-C₆-alkylene, C₃-C₈-cycloalkylene-C₁-C₂-alkylene-C₃-C₈-cycloalkylene or C₁-C₆-alkylene-C₃-C₈-cycloalkylene-C₁-C₆-alkylene; and
h is the number 0 or 1;
(b) fixing the compound of formula (1) onto the material surface using radiation;
(c) reacting the so modified surface with a functional polymerization initiator having a functional group that is co-reactive to L₂ or L₂'; and
(d) applying one or more different ethylenically unsaturated hydrophilic monomers or macromonomers to the bulk material surface obtainable according to step (c) and polymerizing said monomers or macromonomers, thereby providing a surface coating onto the material surface.

2. A process according to claim 1, wherein the radiation is UV or visible light.

3. A process according to claim 1 or 2, wherein the material surface is the surface of a biomedical device, particularly a contact lens, intraocular lens or artificial cornea.

4. A process according to any one of claims 1 to 3, wherein L, is the radical of formula 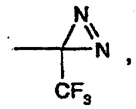 g is 0, and L₂ is carboxy or a derivative thereof or is a radical of formula -L₃-(spacer)-L₂', wherein L₃ is -C(O)O- or -C(O)NH-, (spacer) is linear C₂-C₁₂-alkylene or -(C₂-C₃-alkylene)-O-(CH₂CH₂O)₁₈₋₁₆₀-(C₂-C₃-alkylene)-, and L₂' is carboxy, a carboxy derivative or a radical -O-C(O)-(CH₂)₂-X₂, wherein X₂ is carboxy or a carboxy derivative.

5. A process according to any one of claims 1 to 3, wherein L₁ is the azide radical -N₃, g is 0 or 1, R₂₉ is methyl, methoxy, hydroxy or nitro, and L₂ is amino, carboxy, a carboxy derivative, isocyanato, isothiocyanato or a radical of formula -L₃-(spacer)-L₂', wherein L₃ is -NH- -C(O)O- or -C(O)NH-, (spacer) is linear C₂-C₁₂-alkylene or
-(C₂-C₃-alkylene)-O-(CH₂CH₂O)₁₈₋₁₆₀-(C₂-C₃-alkylene)-, and L₂' is carboxy, a carboxy derivative or a radical -O-C(O)-(CH₂)₂-X₂, wherein X₂ is carboxy or a carboxy derivative.

6. A process according to any one of claims 1 to 3, wherein L₁ is a radical of formula 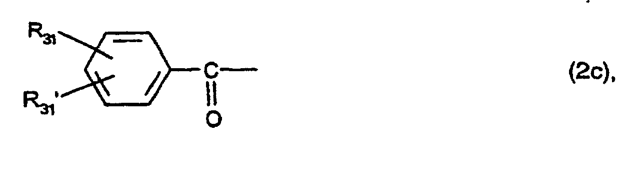 wherein R₃₁ is hydrogen and R₃₁' is hydrogen or amino , or R₃₁ and R₃₁' together are an anhydride radical 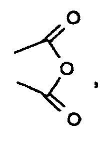 and L₂ is amino, g is 0 or 1 and R₂₉ is amino, or L₂ and R₂₉ together are a radical

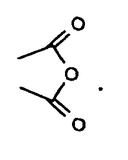

7. A process according to any one of claims 1 to 6, wherein the polymerization initiator according to step (b) is a photoinitiator of formula 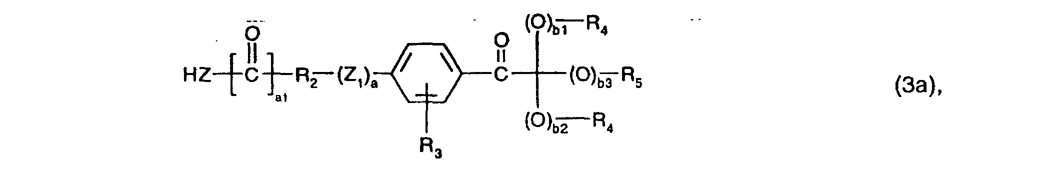 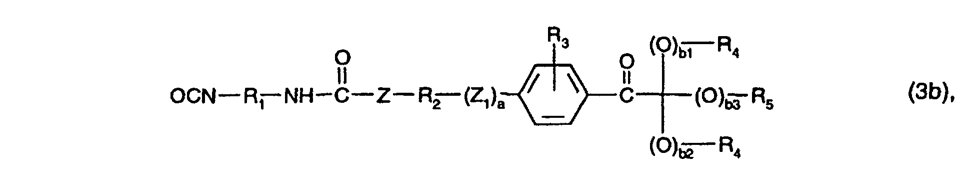 or 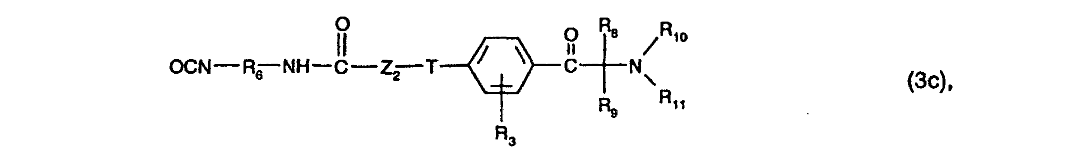 wherein Z is bivalent -O-, -NH- or -NR₁₂-; Z₁ is -O-, -O-(O)C-, -C(O)-O- or -O-C(O)-O-; R₃ is H, C₁-C₁₂-alkyl, C₁-C₁₂-alkoxy or N-C₁-C₁₂-alkylamino; R₄ and R₅ are each independently of the other H, linear or branched C₁-C₈-alkyl, C₁-C₈-hydroxyalkyl or C₆-C₁₀-aryl, or the groups R₄-(O)_{b1}- and R₄-(O)_{b2}- together are -(CH₂)_{c}- wherein c is an integer from 3 to 5, or the groups R₄-(O)_{b1}-, R₄-(O)_{b2}- and R₅-(O₁)_{b3}- together are a radical of the formula 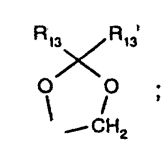 R₂ is a direct bond or linear or branched C₁-C₈-alkylene that is unsubstituted or substituted by -OH and/or is uninterrupted or interrupted by one or more groups -O-, - O-C(O)- or -O-C(O)-O-; R₁ is branched C₃-C₁₈-alkylene, unsubstituted or C₁-C₄-alkyl- or C₁-C₄-alkoxy-substituted C₆-C₁₀-arylene, or unsubstituted or C₁-C₄-alkyl- or C₁-C₄-alkoxy-substituted C₇-C₁₈-aralkylene, unsubstituted or C₁-C₄-alkyl- or C₁-C₄-alkoxy-substituted C₃-C₈-cycloalkylene, unsubstituted or C₁-C₄-alkyl- or C₁-C₄-alkoxy-substituted C₃-C₈-cyclo-alkylene-C_{y}H_{2y}- or unsubstituted or C₁-C₄-alkyl- or C₁-C₄-alkoxy-substituted -C_{y}H_{2y}-(C₃-C₈-cycloalkylene)-C_{y}H_{2y}- wherein y is an integer from 1 to 6; R₆ independently has the same definitions as R₁ or is linear C₃-C₁₈-alkylene; R₁₂ is linear or branched C₁-C₆-alkyl; T is bivalent -O-, -NH-, -S-, C₁-C₈-alkylene or 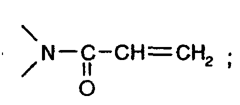 Z₂ is a direct bond or - O-(CH₂)_{d}- or -(OCH₂CH₂)_{d}- wherein d is an integer from 1 to 6 and the terminal CH₂ group of which is each linked to the adjacent T in formula (3c); R₈ is linear or branched C₁-C₈-alkyl, C₂-C₈-alkenyl or C₆-C₁₀-aryl-C₁-C₈-alkyl; R₉ independently of R₈ has the same definitions as R₈ or is C₆-C₁₀-aryl, or R₈ and R₉ together are -(CH₂)ₑ- wherein e is an integer from 2 to 6; R₁₀ and R₁₁ are each independently of the other linear or branched C₁-C₈-alkyl that may be substituted by C₁-C₄-alkoxy, or C₆-C₁₀-aryl-C₁-C₈-alkyl or C₂-C₈-alkenyl; or R₁₀ and R₁₁ together are -(CH₂)_{f1}-Z₃-(CH₂)_{f2}- wherein Z₃ is a direct bond, -O-, -S- or -NR₇-, and R₇ is H or C₁-C₈-alkyl and f1 and f2 are each independently of the other an integer from 2 to 4; R₁₃ and R₁₃' are each independently of the other H, C₁-C₈-alkyl, C₃-C₈-cycloalkyl, benzyl or phenyl; and a, a1, b1, b2 and b3 are each independently of the other 0 or 1; subject to the provisos that b1 and b2 are each 0 when R₁₅ is H; that the total of (b1+b2+b3) is not exceeding 2; and that a is 0 when R₁₂ is a direct bond.

8. A process according to any one of claims 1 to 7, wherein a macromonomer of formula 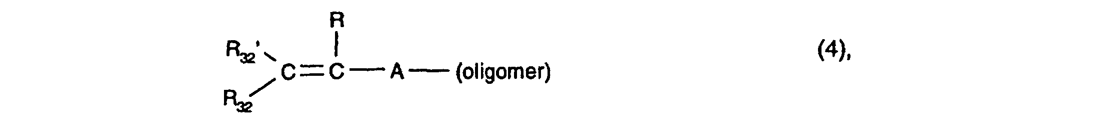 is applied in step (c),
wherein R₃₂ is hydrogen, C₁-C₆-alkyl or a radical -COOR';
R, R' and R₃₂' are each independently of the other hydrogen or C₁-C₆-alkyl;
A is a direct bond or is a radical of formula
-C(O)-(A₁)ₙ-X- (5a)
or
- (A₂)ₘ-NH-C(O)-X- (5b);
or
-(A₂)ₘ-X-C(O)- (5c);
or
- C(O)-NH-C(O)-X- (5d);
or
- C(O)-X₁-(alk*)-X-C(O)- (5e);
or
A and R₃₂, together with the adjacent double bond, are a radical of formula 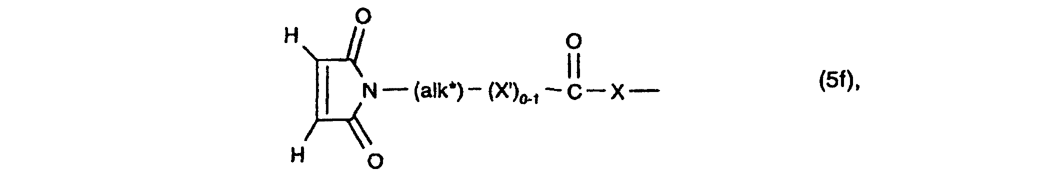 A₁ is -O-C₂-C₁₂-alkylene which is unsubstituted or substituted by hydroxy, or is - O-C₂-C₁₂-alkylene-NH-C(O)- or -O-C₂-C₁₂-alkylene-O-C(O)-NH-R₃₃-NH-C(O)- or - NH-(Alk*)-C(O)-, wherein (Alk*) is C₁-C₆-alkylene and R₃₃ is linear or branched C₁-C₁₈-alkylene or unsubstituted or C₁-C₄-alkyl- or C₁-C₄-alkoxy-substituted C₆-C₁₀-arylene, C₇-C₁₈-aralkylene, C₆-C₁₀-arylene-C₁-C₂-alkylene-C₆-C₁₀-arylene, C₃-C₈-cycloalkylene, C₃-C₈-cycloalkylene-C₁-C₆-alkylene, C₃-C₈-cycloalkylene-C₁-C₂-alkylene-C₃-C₈-cycloalkylene or C₁-C₆-alkylene-C₃-C₈-cycloalkylene-C₁-C₆-alkylene ;
A₂ is C₁-C₈-alkylene; phenylene or benzylene;
m and n are each independently of the other the number 0 or 1;
X, X₁ and X' are each independently of the other a bivalent group -O- or -NR", wherein R" is hydrogen or C₁-C₆-alkyl;
(alk*) is C₂-C₁₂-alkylene;
and (oligomer) denotes
(i) the radical of a telomer of formula 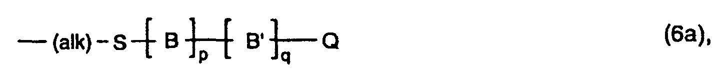 wherein (alk) is C₂-C₁₂-alkylene,
Q is a monovalent group that is suitable to act as a polymerization chain-reaction terminator,
p and q are each independently of another an integer from 0 to 350, wherein the total of (p+q) is an integer from 2 to 350,
and B and B' are each independently of the other a 1,2-ethylene radical derivable from a copolymerizable vinyl monomer by replacing the vinylic double bond by a single bond, at least one of the radicals B and B' being substituted by a hydrophilic substituent; or
(ii) the radical of an oligomer of the formula 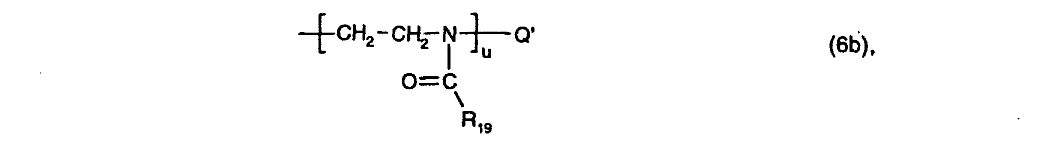 wherein R₁₉ is hydrogen or unsubstituted or hydroxy-substituted C₁-C₁₂-alkyl, u is an integer from 2 to 250 and Q' is a radical of a polymerization initiator; or
(iii) the radical of formula 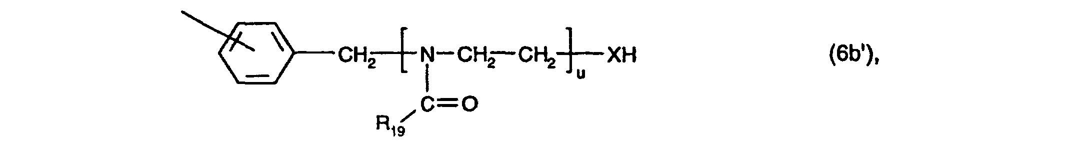 wherein R₁₉, X and u are as defined above, or
(iv) the radical of an oligomer of formula 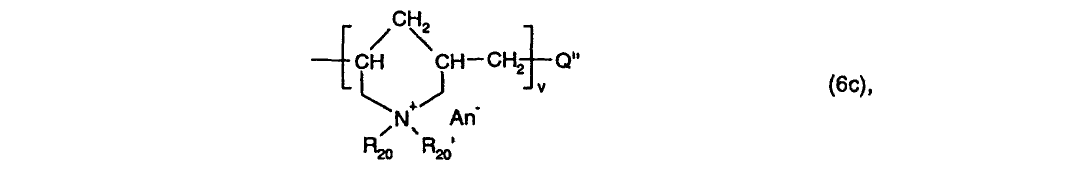 wherein R₂₀ and R₂₀' are each independently C₁-C₄-alkyl, An⁻ is an anion, v is an integer from 2 to 250, and Q" is a monovalent group that is suitable to act as a polymerization chain-reaction terminator; or
(v) the radical of an oligopeptide of formula
- (CHR₂₁-C(O)-NH)ₜCHR₂₁-COOH (6d)
or
-CHR₂₁-(NH-C(O)-CHR₂₁)ₜ-NH₂ (6d'),
or
wherein R₂₁ is hydrogen or C₁-C₄-alkyl which is unsubstituted or substituted by hydroxy, carboxy, carbamoyl, amino, phenyl, o-, m- or p-hydroxyphenyl, imidazolyl, indolyl or a radical -NH-C(=NH)-NH₂ and t is an integer from 2 to 250, or the radical of an oligopeptide based on proline or hydroxyproline; or
(vi) the radical of a polyalkylene oxide of formula
- (alk"-O)_{z} -[CH₂-CH₂-O]ᵣ-[CH₂-CH(CH₃)-O]ₛ-R₃₄ (6e),
wherein R₃₄ is hydrogen or C₁-C₂₄-alkyl, (alk") is C₂-C₄-alkylene, z is 0 or 1, r and s are each independently an integer from 0 to 250 and the total of (r+s) is from 2 to 250; or
(vii) the radical of an oligosaccharide;
subject to the provisos that
A is not a direct bond if (oligomer) is a radical of formula (6a);
A is a radical of formula (5a), (5b) or (5d) or A and R₃₂, together with the adjacent double bond, are a radical of formula (5f) if (oligomer) is a radical of formula (6b), (6c), (6d) or (6e)
or is the radical of an oligosaccharide;
A is a direct bond if (oligomer) is a radical of formula (6b'); and
A is a radical of formula (5c) or (5e) if (oligomer) is a radical of formula (6d').

9. A process according to claim 8, wherein R is hydrogen or methyl, R₃₂ and R₃₂' are each hydrogen, A is a radical of the formula (5a) and (oligomer) is a radical of formula (6a).

10. A process according to claim 8 or 9, wherein (oligomer) is a radical of formula 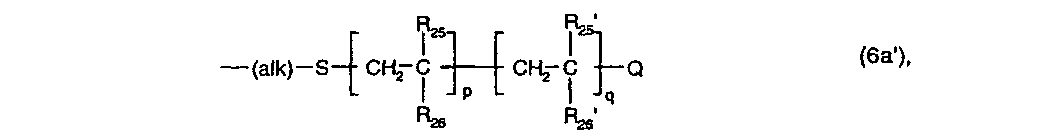 wherein (alk) is C₂-C₄-alkylene, R₂₅ and R₂₅' are each independently hydrogen or methyl, Q is a monovalent group that is suitable to act as a polymerization chain-reaction terminator, p and q are each independently an integer from 0 to 100 wherein the total of (p+q) is an integer from 5 to 100, and R₂₆ and R₂₆' are each independently a radical -COOY, wherein Y is C₁-C₂-alkyl, C₂-C₃-alkyl, which is substituted by hydroxy, amino or N,N-di-C₁-C₂-alkyl-amino, or is a radical -C₂-C₄-alkylene-NH-C(O)-O-G wherein -O-G is the radical of trehalose; a radical -CO-NY₁Y₂, wherein Y₁ and Y₂ are each independently of the other hydrogen or C₁-C₂-alkyl which is unsubstituted or substituted by hydroxy, or Y₁ and Y₂ together with the adjacent N-atom form a N-C₁-C₂-alkylpiperazino or morpholino ring; a heterocyclic radical selected from the group consisting of N-pyrrolidonyl, 2- or 4-pyridinyl, 2-methylpyridin-5-yl, 2-, 3- oder 4-hydroxypyridinyl, N-ε-caprolactamyl, N-imidazolyl, 2-methylimidazol-1-yl, N-morpholinyl and 4-N-methyipiperazin-1-yl; -COOH; -SO₃H; o-, m- or p-sulfophenyl; o-, m- or p-sulfomethylphenyl; a radical -CONY₅Y₆ wherein Y₅ is C₂-C₄-alkyl substituted by sulfo, and Y₆ is hydrogen; C₁-C₄-alkyl which is substituted by -NR₂₃R₂₃'R₂₃"⁺An⁻ wherein R₂₃, R₂₃' and R₂₃" are each independently of another hydrogen or C₁-C₄-alkyl and An⁻ is an anion; a -C(O)OY₇ wherein Y₇ is C₂-C₄-alkyl, which is substituted by -NR₂₃R₂₃'R₂₃"⁺An⁻ and is further unsubstituted or substituted by hydroxy, wherein R₂₃, R₂₃', R₂₃" and ⁺An⁻ are as defined; and a radical -C(O)O-CH₂-CH(OY₈)-CH₂-O-PO₂⁻-(CH₂)₂-N(CH₃)₃⁺, wherein Y₈ is hydrogen or the acyl radical of a higher fatty acid.

11. A process according to any one of claims 1 to 10, wherein in step (c) a macromonomer of formula 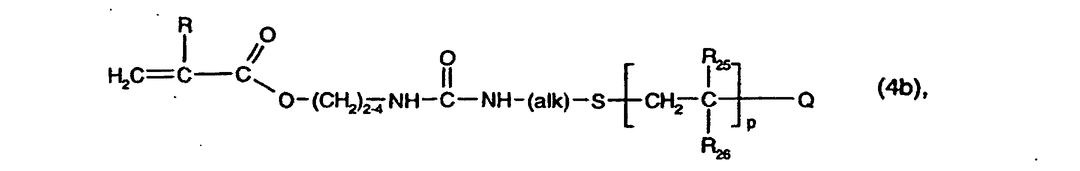 is applied, wherein R is hydrogen or methyl, (alk) is C₂-C₄-alkylene, R₂₅ is hydrogen or methyl, p is an integer of 5 to 50, Q is a monovalent group that is suitable to act as a polymerization chain-reaction terminator, and R₂₆ is a radical -CONH₂, -CON(CH₃)₂ or

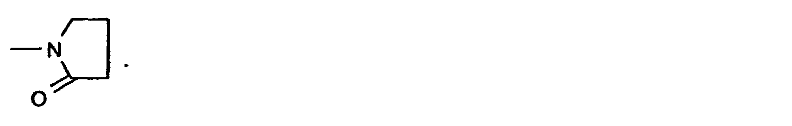

12. A process according to claim 1 comprising the steps of:
(a) applying to the the material surface a compound of formula 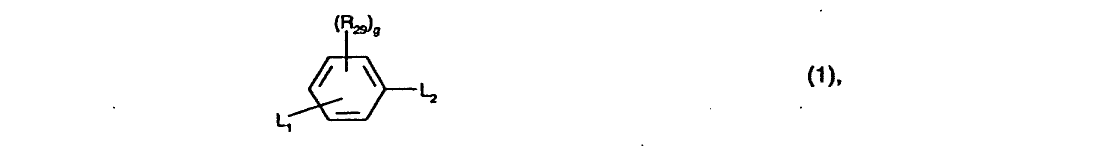 wherein g is 0 or 1, R₂₉ is methyl, methoxy, hydroxy or nitro, L₁ is the azide radical -N₃, and
L₂ is amino, carboxy, a carboxy derivative, isocyanato or isothiocyanato;
(b) fixing the compound of formula (1) onto the material surface using radiation;
(c) reacting the so modified surface with a functional polymerization initiator having a functional group that is co-reactive to L₂; and
(d) applying a hydrophilic macromonomer of the formula 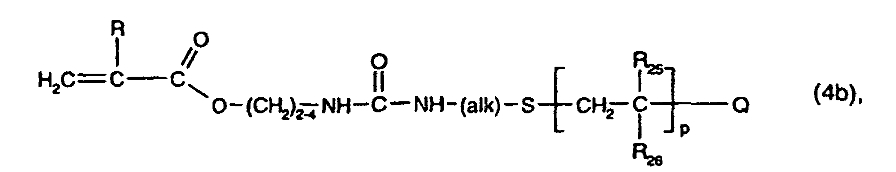 wherein R and R₂₅ are each independently hydrogen or methyl, (alk) is 1,2-ethylene, R₂₆ is -CONH₂, -CON(CH₃)₂ or 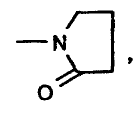 p is an integer of from 5 to 250, and Q is a monovalent group that is suitable to act as a polymerization chain-reaction terminator, to the bulk material surface obtainable according to step (b) and polymerizing said macromonomer, thereby providing a surface coating onto the material surface.

13. A process according to any one of claims 1 to 12, wherein the material surface is the surface of a biomedical device, in particular of a contact lens, intraocular lens or artificial cornea.

## Patentansprüche

1. Verfahren zum Beschichten einer Materialoberfläche, umfassend die folgenden Stufen:
(a) Aufbringen auf die Materialoberfläche einer Verbindung der Formel 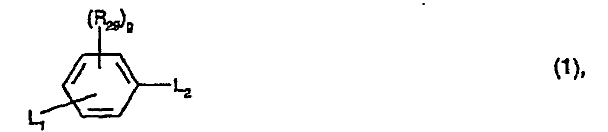 worin R₂₉ C₁-C₄-Alkyl, C₁-C₄-Alkoxy, Hydroxy, Sulfo, Nitro, Trifluormethyl oder Halogen bedeutet,
g eine ganze Zahl von 0 bis 2 ist,
L₁ eine Gruppe ist, die als triggerierbarer Vorläufer für die Carben-, Nitren- oder Benzhydrol-Bildung fungiert,
L₂ Amino, C₁-C₄-Alkylamino, Hydroxy, Glycidyl, Carboxy oder ein Derivat hiervon,
Isocyanato oder Isothiocyanato bedeutet, oder ein Rest der Formel
- [L₃]ₕ - (spacer) - L₂' (1a),
ist, oder
L₂ und R₂₉ gemeinsam einen Anhydridrest 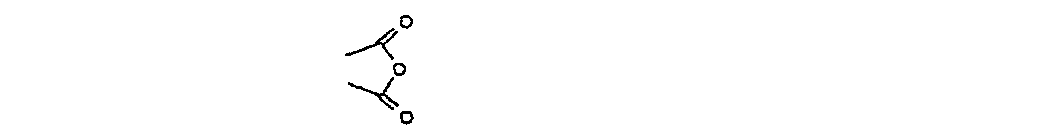 bilden;
L₂' Amino, C₁-C₄-Alkylamino, Hydroxy, Carboxy oder ein Derivat hiervon, Isocyanato,
Isothiocyanato, -O-Glycidyl oder -O-C(O)-(CH₂)ₕ₁-X₂ bedeutet, worin h1 für 1 bis 4 steht
und X₂ Carboxy oder ein Derivat hiervon ist,
L₃ für -NH-, -NC₁-C₆-Alkyl-, -O-, -C(O)O-, -C(O)NH-, -NHC(O)NH-, -NHC(O)O- oder -OC(O)NH- steht;
(spacer) lineares oder verzweigtes C₁-C₂₀₀-Alkylen ist, das durch Hydroxy substituiert und/oder mit Ausnahme von C₁-Alkyl durch -O- unterbrochen sein kann, oder C₃-C₈-Cycloalkylen, C₃-C₈-Cycloalkylen-C₁-C₆-alkylen, C₃-C₈-Cycloalkylen-C₁-C₂-alkylen-C₃-C₈-cycloalkylen oder C₁-C₆-Alkylen-C₃-C₈-cycloalkylen-C₁-C₆-alkylen bedeutet; und h für die Zahl 0 oder 1 steht;
(b) Fixieren der Verbindung der Formel (1) auf der Materialoberfläche unter Anwendung von Strahlung;
(c) Umsetzen der so modifizierten Oberfläche mit einem funktionellen Polymerisations-Initiator mit einer funktionellen Gruppe, die in Bezug auf L₂ oder L₂' co-reaktiv ist; und
(d) Aufbringen von einem oder mehreren verschiedenen ethylenisch ungesättigten hydrophilen Monomeren oder Makromonomeren auf die gemäß Stufe (c) erhältliche Materialoberflächen-Masse und Polymerisieren dieser Monomeren oder Makromonomeren, wodurch eine Oberflächenbeschichtung auf der Materialoberfläche gebildet wird.

2. Verfahren gemäß Anspruch 1, worin die Strahlung UV oder sichtbares Licht ist.

3. Verfahren gemäß Anspruch 1 oder 2, worin die Materialoberfläche die Oberfläche einer biomedizinischen Vorrichtung, insbesondere einer Kontaktlinse, Intraokularlinse oder künstlichen Cornea ist.

4. Verfahren gemäß einem der Ansprüche 1 bis 3, worin L₁ der Rest der Formel 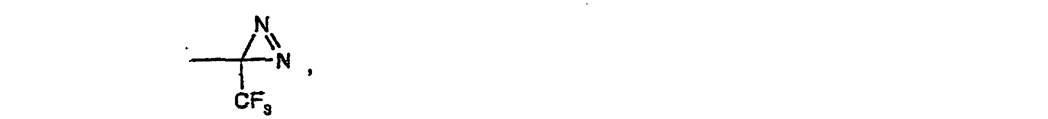 ist, g für 0 steht, und L₂ Carboxy oder ein Derivat hiervon ist, oder ein Rest der Formel -L₃-(spacer)-L₂' ist, worin L₃ für -C(O)O- oder -C(O)NH- steht, (spacer) lineares C₂-C₁₂-Alkylen oder -(C₂-C₃-Alkylen)-O-(CH₂CH₂O)₁₈₋₁₆₀-(C₂-C₃-alkylen)- bedeutet, und L₂' Carboxy, ein Carboxyderivat oder ein Rest -O-C(O)-(CH₂)₂-X₂ ist, worin X₂ Carboxy oder ein Carboxyderivat ist.

5. Verfahren gemäß einem der Ansprüche 1 bis 3, worin L₁ der Azidrest -N₃ ist, g für 0 oder 1 steht, R₂₉ Methyl, Methoxy, Hydroxy oder Nitro ist, und L₂ Amino, Carboxy, ein Carboxyderivat, Isocyanato, Isothiocyanato oder ein Rest der Formel -L₃-(spacer)-L₂' ist, worin L₃ für -NH-, -C(O)O- oder -C(O)NH- steht, (spacer) lineares C₂-C₁₂-Alkylen oder -(C₂-C₃-Alkylen)-O-(CH₂CH₂O)₁₈₋₁₆₀-(C₂-C₃-alkylen)- ist, und L₂' Carboxy, ein Carboxyderivat oder einen Rest -O-C(O)-(CH₂)₂-X₂ bedeutet, worin X₂ Carboxy oder ein Carboxyderivat ist.

6. Verfahren gemäß einem der Ansprüche 1 bis 3, worin L₁ ein Rest der Formel 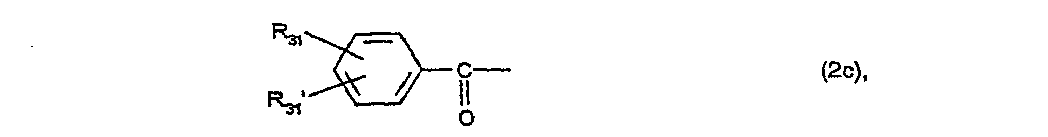 ist, worin R₃₁ Wasserstoff bedeutet und R₃₁' Wasserstoff oder Amino ist, oder R₃₁ und R₃₁' gemeinsam ein Anhydridrest 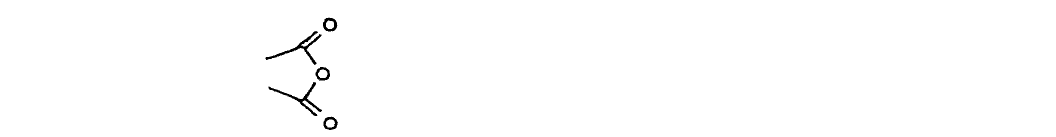 sind, und L₂ Amino ist, g für 0 oder 1 steht und R₂₉ Amino ist, oder L₂ und R₂₉ gemeinsam einen Rest 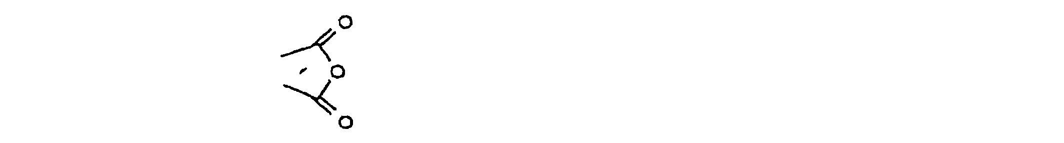 bilden.

7. Verfahren gemäß einem der Ansprüche 1 bis 6, worin der Polymerisations-Initiator gemäß Stufe (b) ein Photo-Initiator der Formel 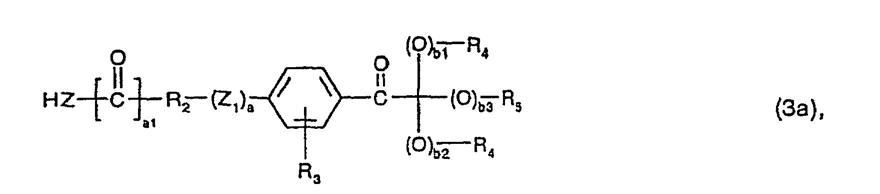 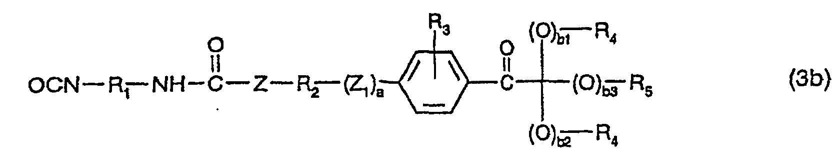 oder 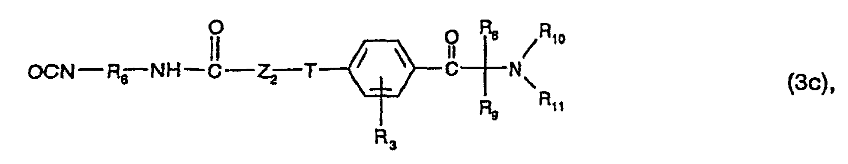 ist, worin Z bivalentes -O-, -NH- oder -NR₁₂- ist; Z₁ für -O-, -O-(O)C-, -C(O)-O- oder -O-C(O)-O- steht; R₃ H, C₁-C₁₂-Alkyl, C₁-C₁₂-Alkoxy oder N-C₁-C₁₂-Alkylamino ist; R₄ und R₅ jeweils unabhängig voneinander H, lineares oder verzweigtes C₁-C₈-Alkyl, C₁-C₈-Hydroxyalkyl oder C₆-C₁₀-Aryl sind, oder die Gruppen R₄-(O)_{b1}- und R₄-(O)_{b2}- gemeinsam -(CH₂)_{c}- sind, worin c eine ganze Zahl von 3 bis 5 ist, oder die Gruppen R₄-(O)_{b1}-, R₄-(O)_{b2}- und R₅-(O₁)_{b3}- gemeinsam ein Rest der Formel 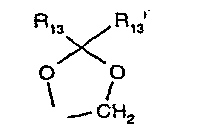 sind; R₂ eine direkte Bindung oder lineares oder verzweigtes C₁-C₈-Alkylen ist, das unsubstituiert oder substituiert ist durch -OH und/oder nicht-unterbrochen oder unterbrochen ist durch eine oder mehrere Gruppen -O-, -O-C(O)- oder -O-C(O)-O-; R₁ verzweigtes C₃-C₁₈-Alkylen, unsubstituiertes oder C₁-C₄-Alkyl- oder C₁-C₄-Alkoxy-substituiertes C₆-C₁₀-Arylen, oder unsubstituiertes oder C₁-C₄-Alkyl- oder C₁-C₄-Alkoxy-substituiertes C₇-C₁₈-Aralkylen, unsubstituiertes oder C₁-C₄-Alkyl- oder C₁-C₄-Alkoxy-substituiertes C₃-C₈-Cycloalkylen, unsubstituiertes oder C₁-C₄-Alkyl- oder C₁-C₄-Alkoxy-substituiertes C₃-C₈-Cycloalkylen-C_{y}H_{2y}- oder unsubstituiertes oder C₁-C₄-Alkyl- oder C₁-C₄-Alkoxy-substituiertes -C_{y}H_{2y}-(C₃-C₈-Cycloalkylen)-C_{y}H_{2y}- ist, worin y eine ganze Zahl von 1 bis 6 ist; R₆ unabhängig die gleichen Definitionen wie R₁ besitzt oder lineares C₃-C₁₈-Alkylen ist; R₁₂ lineares oder verzweigtes C₁-C₆-Alkyl ist; T bivalentes -O-, -NH-, -S-, C₁-C₈-Alkylen oder 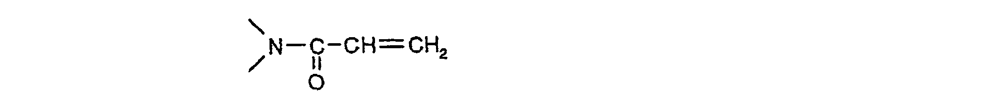 ist; Z₂ eine direkte Bindung oder -O-(CH₂)_{d}- oder -(OCH₂CH₂)_{d}- ist, worin d eine ganze Zahl von 1 bis 6 ist und die endständige CH₂-Gruppe hiervon jeweils an das benachbarte T in Formel (3c) gebunden ist; R₈ lineares oder verzweigtes C₁-C₈-Alkyl, C₂-C₈-Alkenyl oder C₆-C₁₀-Aryl-C₁-C₈-alkyl ist; R₉ unabhängig von R₈ die gleichen Definitionen wie R₈ besitzt oder C₆-C₁₀-Aryl ist, oder R₈ und R₉ gemeinsam -(CH₂)ₑ- sind, worin e eine ganze Zahl von 2 bis 6 ist; R₁₀ und R₁₁ jeweils unabhängig voneinander lineares oder verzweigtes C₁-C₈-Alkyl, das substituiert sein kann durch C₁-C₄-Alkoxy, oder C₆-C₁₀-Aryl-C₁-C₈-alkyl oder C₂-C₈-Alkenyl sind; oder R₁₀ und R₁₁ gemeinsam -(CH₂)_{f1}-Z₃-(CH₂)_{f2}- sind, worin Z₃ eine direkte Bindung, -O-, -S- oder -NR₇- ist, und R₇ für H oder C₁-C₈-Alkyl steht und f1 und f2 jeweils unabhängig voneinander eine ganze Zahl von 2 bis 4 sind; R₁₃ und R₁₃' jeweils unabhängig voneinander H, C₁-C₈-Alkyl, C₃-C₈-Cycloalkyl, Benzyl oder Phenyl sind; und a, al, b1, b2 und b3 jeweils unabhängig voneinander 0 oder 1 sind; mit der Maßgabe, dass b1 und b2 jeweils 0 sind, wenn R₁₅ für H steht; dass die Summe von (b1+b2+ b3) 2 nicht überschreitet; und dass a für 0 steht, wenn R₁₂ eine direkte Bindung ist.

8. Verfahren gemäß einem der Ansprüche 1 bis 7, worin ein Makromonomeres der Formel 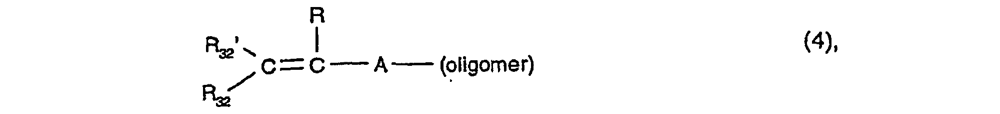 in Stufe (c) aufgebracht wird, worin
R₃₂ Wasserstoff, C₁-C₆-Alkyl oder ein Rest -COOR' ist; worin R, R' und R₃₂' jeweils unabhängig voneinander Wasserstoff oder C₁-C₆-Alkyl sind;
A eine direkte Bindung ist oder ein Rest der Formel
- C(O)-(A₁)ₙ-X- (5a)
oder
-(A₂)ₘ-NH-C(O)-X- (5b);
oder
-(A₂)ₘ-X-C(O)- (5c);
oder
-C(O)-NH-C(O)-X- (5d);
oder
-C(O)-X₁-(alk*)-X-C(O)- (5e);
ist, oder A und R₃₂ gemeinsam mit der benachbarten Doppelbindung ein Rest der Formel 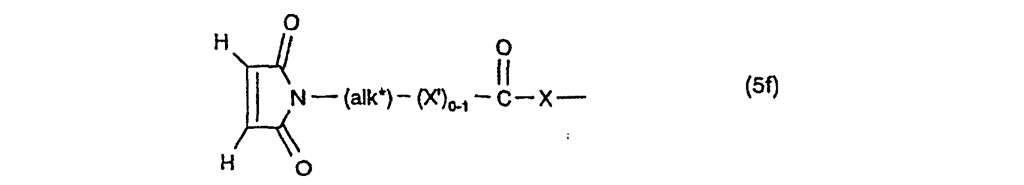 sind,
A ₁ -O-C₂-C₁₂-Alkylen ist, das unsubstituiert ist oder substituiert durch Hydroxy, oder -O-C₂-C₁₂-Alkylen-NH-C(O)- oder -O-C₂-C₁₂-Alkylen-O-C(O)-NH-R₃₃-NH-C(O)- oder -NH-(Alk*)-C(O)- ist, worin (Alk*) C₁-C₆-Alkylen ist und R₃₃ lineares oder verzweigtes C₁-C₁₈-Alkylen oder unsubstituiertes oder C₁-C₄-Alkyl- oder C₁-C₄-Alkoxy-substituiertes C₆-C₁₀-Arylen, C₇-C₁₈-Aralkylen, C₆-C₁₀-Arylen-C₁-C₂-alkylen-C₆-C₁₀-arylen, C₃-C₈-Cycloalkylen, C₃-C₈-Cycloalkylen-C₁-C₆-alkylen, C₃-C₈-Cycloalkylen-C₁-C₂-alkylen-C₃-C₈cycloalkylen oder C₁-C₆-Alkylen-C₃-C₈-cycloalkylen-C₁-C₆-alkylen ist;
A₂ C₁-C₈-Alkylen; Phenylen oder Benzylen ist;
m und n jeweils unabhängig voneinander die Zahl 0 oder 1 bedeuten;
X, X₁ und X' jeweils unabhängig voneinander eine zweiwertige Gruppe -O- oder -NR" sind, worin R" Wasserstoff oder C₁-C₆-Alkyl ist;
(alk*) C₂-C₁₂-Alkylen ist;
und (oligomer) bezeichnet
(i) den Rest eines Telomeren der Formel 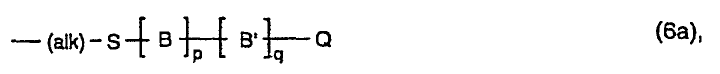 worin (alk) C₂-C₁₂-Alkylen bedeutet,
Q eine einwertige Gruppe ist, die imstande ist, als Polymerisationskettenreaktions-Terminator zu reagieren,
p und q jeweils unabhängig voneinander eine ganze Zahl von 0 bis 350 sind, worin die Summe von (p+q) eine ganze Zahl von 2 bis 350 ist,
und B und B'jeweils unabhängig voneinander ein 1,2-Ethylenrest sind, der herleitbar ist von einem copolymerisierbaren Vinylmonomeren durch Austausch der vinylischen Doppelbindung durch eine Einfachbindung, wobei zumindest einer der Reste B und B' durch einen hydrophilen Substituenten substituiert wird; oder
(ii) den Rest eines Oligomeren der Formel 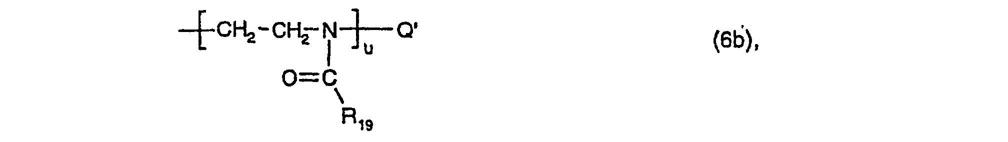 worin R₁₉ Wasserstoff oder unsubstituiertes oder durch Hydroxy substituiertes C₁-C₁₂-Alkyl ist, u eine ganze Zahl von 2 bis 250 ist und Q' ein Rest eines PolymerisationsInitiators ist; oder
(iii) den Rest der Formel 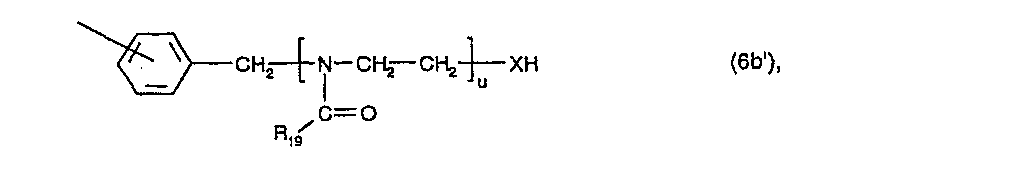 worin R₁₉, X und u wie vorstehend definiert sind, oder
(iv) den Rest eines Oligomeren der Formel 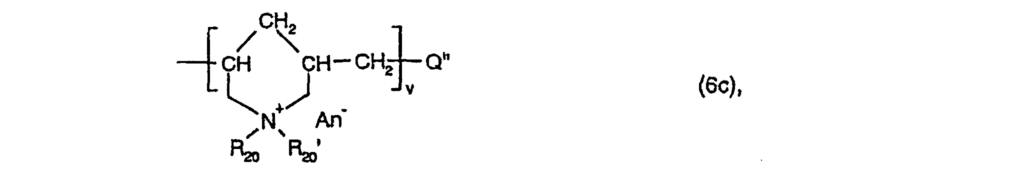 worin R₂₀ und R₂₀' jeweils unabhängig C₁-C₄-Alkyl sind An⁻ ein Anion ist, v eine ganze Zahl von 2 bis 250 ist, und Q" eine einwertige Gruppe ist, die geeignet ist, als Polymerisationskettenreaktions-Terminator zu reagieren, oder
(v) den Rest eines Oligopeptids der Formel
- (CHR₂₁-C(O)-NH)ₜ-CHR₂₁-COOH (6d)
oder
- CHR₂₁-(NH-C(O)-CHR₂₁)ₜ-NH₂ (6d')
worin R₂₁ Wasserstoff oder C₁-C₄-Alkyl ist, das unsubstituiert ist oder substituiert ist durch Hydroxy, Carboxy, Carbamoyl, Amino, Phenyl, o-, m- oder p-Hydroxyphenyl, Imidazolyl, Indolyl oder einen Rest -NH-C(=NH)-NH₂, und t eine ganze Zahl von 2 bis 250 ist, oder den Rest eines auf Prolin oder Hydroxyprolin basierenden Oligopeptids; oder
(vi) den Rest eines Polyalkylenoxids der Formel
-(alk"-O)_{z} -[CH₂-CH₂-O]ᵣ-[CH₂-CH(CH₃)-O]ₛ-R₃₄ (6e),
worin R₃₄ Wasserstoff oder C₁-C₂₄-Alkyl, (alk**) C₂-C₄-Alkylen ist, z für 0 oder 1 steht, r und s jeweils unabhängig eine ganze Zahl von 0 bis 250 sind und die Summe von (r+s) von 2 bis 250 beträgt; oder
(vii) der Rest eines Oligosaccharids;
mit den Maßgaben, dass
A nicht eine direkte Bindung ist, wenn (oligomer) ein Rest der Formel (6a) ist;
A ein Rest der Formel (5a), (5b) oder (5d) ist oder A und R₃₂, gemeinsam mit der benachbarten Doppelbindung, einen Rest der Formel (5f) bilden, wenn (oligomer) ein Rest der Formel (6b), (6c), (6d) oder (6e) ist oder der Rest eines Oligosaccharids ist;
A eine direkte Bindung ist, wenn (oligomer) ein Rest der Formel (6b') ist; und
A ein Rest der Formel (5c) oder (5e) ist, wenn (oligomer) ein Rest der Formel (6d') ist.

9. Verfahren gemäß Anspruch 8, worin R Wasserstoff oder Methyl ist. R₃₂ und R₃₂' jeweils Wasserstoff sind, A ein Rest der Formel (5a) ist und (oligomer) ein Rest der Formel (6a) ist.

10. Verfahren gemäß Anspruch 8 oder 9, worin (oligomer) ein Rest der Formel 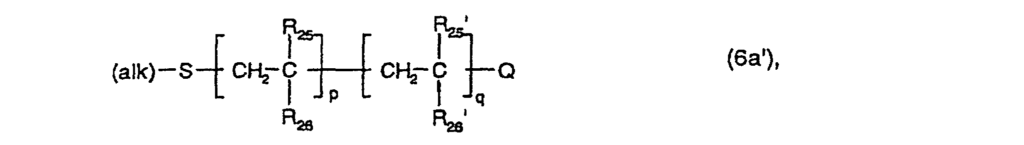 worin (alk) C₂-C₄-Alkylen ist, R₂₅ und R₂₅' jeweils unabhängig voneinander Wasserstoff oder Methyl sind, Q eine einwertige Gruppe ist, die imstande ist, als Polymerisationskettenreaktions-Terminator zu reagieren, p und q jeweils unabhängig voneinander eine ganze Zahl von 0 bis 100 sind, worin die Summe von (p+q) eine ganze Zahl von 5 bis 100 ist, und R₂₆ und R₂₆' jeweils unabhängig ein Rest -COOY sind, worin Y C₁-C₂-Alkyl, C₂-C₃-Alkyl, das substituiert ist durch Hydroxy, Amino oder N,N-Di-C₁-C₂-alkylamino, ist, oder ein Rest -C₂-C₄-Alkylen-NH-C(O)-O-G ist, worin -O-G der Rest von Trehalose ist; ein Rest -CO-NY₁Y₂, worin Y₁ und Y₂ jeweils unabhängig voneinander Wasserstoff oder C₁-C₂-Alkyl sind, das unsubstituiert oder substituiert ist durch Hydroxy, oder Y₁ und Y₂ gemeinsam mit dem benachbarten N-Atom einen N-C₁-C₂-Alkylpiperazino- oder -morpholinoring bilden; ein heterocyclischer Rest, ausgewählt aus der Gruppe, bestehend aus N-Pyrrolidonyl, 2- oder 4-Pyridinyl, 2-Methylpyridin-5-yl, 2-, 3- oder 4-Hydroxypyridinyl, N-ε-Caprolactamyl, N-Imidazolyl, 2-Methylimidazol-1-yl, N-Morpholinyl und 4-N-Methylpiperazin-1-yl; -COOH; -SO₃H; o-, m- oder p-Sulfophenyl; o-, m- oder p-Sulfomethylphenyl; ein Rest -CONY₅Y₆, worin Y₅ durch Sulfo substituiertes C₂-C₄-Alkyl ist, und Y₆ Wasserstoff ist; C₁-C₄-Alkyl, das substituiert ist durch -NR₂₃R₂₃'R₂₃"⁺An⁻, worin R₂₃, R₂₃' und R₂₃" jeweils unabhängig voneinander Wasserstoff oder C₁-C₄-Alkyl sind und An⁻ ein Anion ist; ein Rest -C(O)OY₇, worin Y₇ C₂-C₄-Alkyl ist, das substituiert ist durch -NR₂₃R₂₃'R₂₃"⁺An⁻ und weiterhin unsubstituiert oder substituiert ist durch Hydroxy, worin R₂₃, R₂₃', R₂₃" und ⁺An⁻ die angegebene Bedeutung besitzen; und ein Rest -C(O)O-CH₂-CH(OY₈)-CH₂-O-PO₂-(CH₂)₂-N(CH₃)₃⁺, worin Y₈ Wasserstoff oder der Acylrest einer höheren Fettsäure ist, ist.

11. Verfahren gemäß einem der Ansprüche 1 bis 10, worin in Stufe (e) ein Makromonomeres der Formel 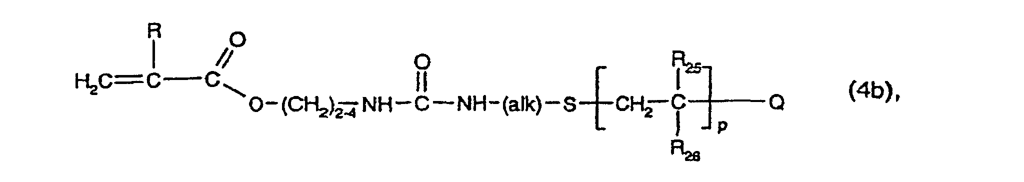 aufgebracht wird, worin R Wasserstoff oder Methyl ist, (alk) C₂-C₄-Alkylen ist, R₂₅ Wasserstoff oder Methyl ist, p eine ganze Zahl von 5 bis 50 ist, Q eine einwertige Gruppe ist, die imstande ist, als Polymerisationskettenreaktions-Terminator zu reagieren, und R₂₆ ein Rest -CONH₂, -CON(CH₃)₂ oder 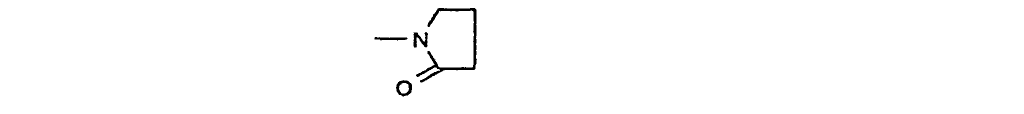 ist.

12. Verfahren gemäß Anspruch 1, umfassend die folgenden Stufen:
(a) Aufbringen auf die Materialoberfläche einer Verbindung der Formel 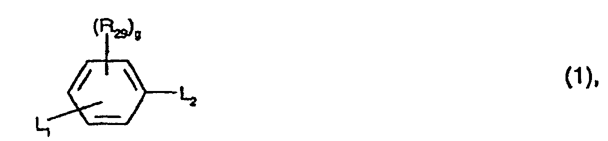 worin g für 0 oder 1 steht, R₂₉ Methyl, Methoxy, Hydroxy oder Nitro ist, L₁ der Azidrest -N₃ ist und L₂ Amino, Carboxy, ein Carboxyderivat, Isocyanato oder Isothiocyanato ist;
(b) Fixieren der Verbindung der Formel (1) auf der Materialoberfläche unter Anwendung von Strahlung;
(c) Umsetzung der so modifizierten Oberfläche mit einem funktionellen Polymerisations-Initiator mit einer funktionellen Gruppe, die im Hinblick auf L₂ co-reaktiv ist; und
(d) Aufbringen eines hydrophilen Makromonomeren der Formel 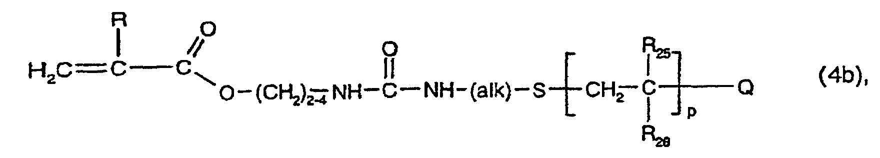
worin R und R₂₅ jeweils unabhängig Wasserstoff oder Methyl sind, (alk) 1,2-Ethylen ist, R₂₆ -CONH₂, -CON(CH₃)₂ oder 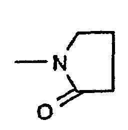 ist, p eine ganze Zahl von 5 bis 250 ist, und Q eine einwertige Gruppe ist, die imstande ist, als Polymerisationskettenreaktions-Terminator zu reagieren, auf die gemäß Stufe (b) erhältliche Materialoberflächen-Masse und Polymerisieren dieses Makromonomeren, wodurch auf der Materialoberfläche eine Oberflächenbeschichtung gebildet wird.

13. Verfahren gemäß einem der Ansprüche 1 bis 12, worin die Materialoberfläche die Oberfläche einer biomedizinischen Vorrichtung, insbesondere Kontaktlinse, Intraokularlinse oder künstlichen Cornea ist.

## Revendications

1. Procédé de recouvrement d'une surface de matériau comprenant les étapes suivantes :
a) application sur la surface du matériau d'un composé de formule 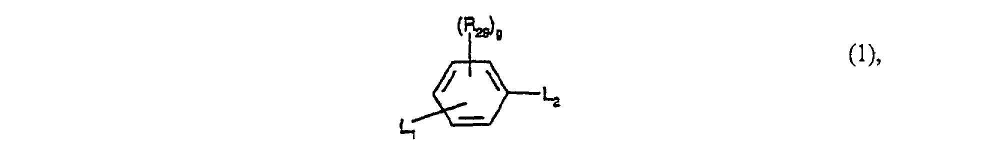 dans laquelle R₂₉ est C₁-C₄-alkyle, C₁-C₄-alcoxy, hydroxy, sulfo, nitro, trifluorométhyle ou halogène,
g est un nombre entier de 0 à 2,
L₁ est un groupe agissant comme précurseur de déclenchement pour la formation de carbène, nitrène ou benzhydrol,
L₂ est amino, C₁-C₄-alkylamino, hydroxy, glycidyle, carboxy ou un de ses dérivés, isocyanate ou isothiocyanate, ou un radical de formule
- [L₃]ₕ-(espaceur)-L₂' (1a),
ou L₂ et R₂₉ forment ensemble un radical anhydride 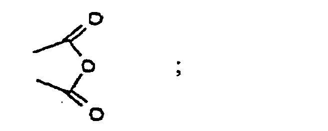 L₂' est amino, C₁-C₄-alkylamino, hydroxy, carboxy ou un de ses dérivés, isocyanate, isothiocyanate, -O-glycidyle ou - O-C-(O)-(CH₂)ₕ₁-X₂, dans lequel hl va de 1 à 4 et X₂ est carboxy ou un de ses dérivés,
L₃ est -NH-, -NC₁-C₆-alkyl-, -O-, -C(O)O-, -C(O)NH-, -NHC(O)NH-, -NHC(O)O- ou -OC(O)NH- ;
(espaceur) est C₁-C₂₀₀-alkylène linéaire ou ramifié pouvant être substitué par hydroxy et/ou interrompu par -O- sauf pour C₁-alkyle, ou est C₃-C₈-cycloalkylène, C₃-C₈-cycloalkylène-C₁-C₆-alkylène, C₃-C₈-cycloalkylène-C₁-C₂-alkylène-C₃-C₈-cycloalkylène ou C₁-C₆-alkylène-C₃-C₈-cycloalkylène-C₁-C₆-alkylène ; et h est le nombre 0 ou 1 ;
b) fixation du composé de formule (1) sur la surface du matériau à l'aide d'un rayonnement ;
c) mise en réaction de la surface ainsi modifiée à l'aide d'un initiateur fonctionnel de polymérisation ayant un groupe fonctionnel qui coréagit avec L₂ ou L₂' ; et
d) application d'un ou plusieurs monomères ou macromonomères hydrophiles à insaturation éthylénique différents sur la surface du matériau brut obtenu conformément à l'étape (c) et polymérisation desdits monomères ou macromonomères procurant ainsi une surface de recouvrement sur la surface du matériau.

2. Procédé selon la revendication 1, **caractérisé en ce que** le rayonnement est une lumière UV ou une lumière visible.

3. Procédé selon la revendication 1 ou 2, **caractérisé en ce que** la surface du matériau est la surface d'un dispositif biomédical, plus particulièrement d'une lentille de contact, d'une lentille intraoculaire ou d'une cornée artificielle.

4. Procédé selon l'une des revendications 1 à 3, **caractérisé en ce que** L₁ est un radical de formule 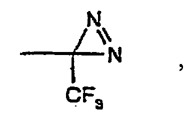 g est 0 et L₂ est carboxy ou l'un de ses dérivés ou est un radical de formule -L₃-(espaceur)-L_{2'}, dans laquelle L₃ est - C(O)O- ou -C(O)NH-, (espaceur) est C₂-C₁₂-alkylène linéaire ou -(C₂-C₃-alkylène)-O-(CH₂CH₂O)₁₈₋₁₆₀-(C₂-C₃-alkylène)- et L₂' est carboxy, un dérivé carboxy ou un radical -O-C(O)-(CH₂)₂-X₂, dans lequel X₂ est carboxy ou un dérivé carboxy.

5. Procédé selon l'une des revendications 1 à 3, **caractérisé en ce que** L₁ est un radical azide -N₃, g est 0 ou 1, R₂₉ est méthyle, méthoxy, hydroxy ou nitro, et L₂ est amino, carboxy, un dérivé de carboxy, isocyanate, isothiocyanate ou un radical de formule L₃-(espaceur)-L₂', dans laquelle L₃ est -NH-, -C(O)O- ou -C(O)NH-, (espaceur) est C₂-C₁₂-alkylène linéaire ou -(C₂-C₃-alkylène)-O-(CH₂CH₂O)₁₈₋₁₆₀-(C₂-C₃-alkylène)- et L₂' est carboxy, un dérivé carboxy ou un radical -O-C(O)-(CH₂)₂-X₂, dans lequel X₂ est carboxy ou un dérivé carboxy.

6. Procédé selon l'une des revendications 1 à 3, dans laquelle L₁ est un radical de formule 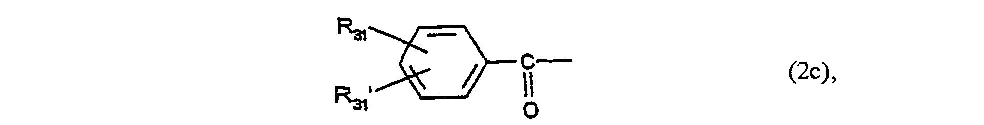 dans laquelle, R₃₁ est hydrogène et R₃₁' est hydrogène ou amino, ou R₃₁ et R₃₁' forment ensemble un radical anhydride 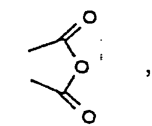 et L2 est amino, g est 0 ou 1 et R₂₉ est amino, ou L₂ et R₂₉ forment ensemble un radical

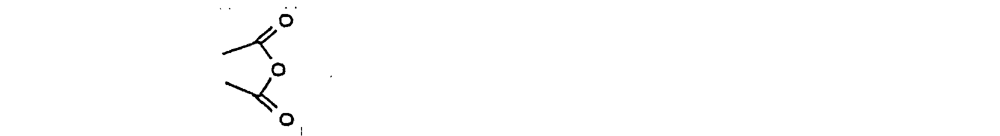

7. Procédé selon l'une des revendications 1 à 6, **caractérisé en ce que** l'initiateur de polymérisation, conformément à l'étape (b), est un photoinitiateur de formule 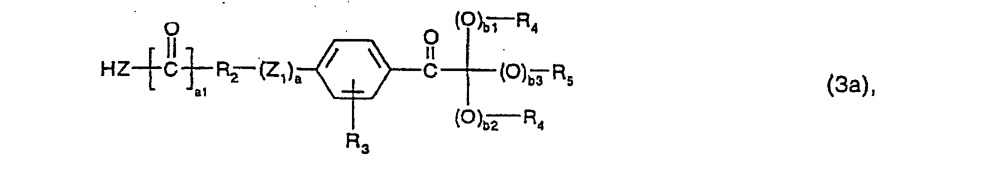 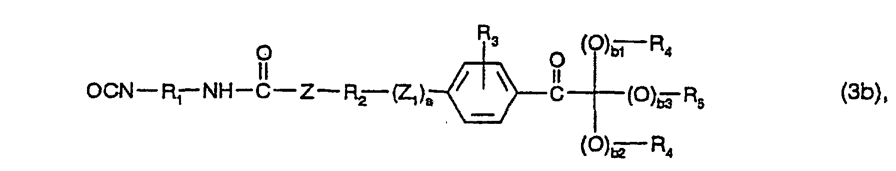 ou 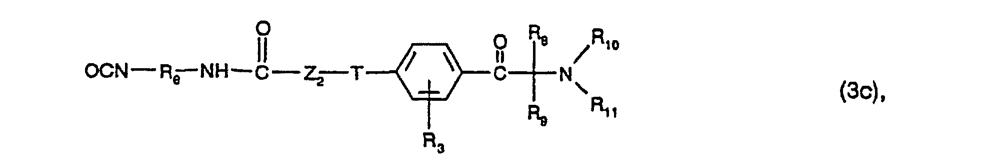 dans laquelle Z est -O-, -NH- ou -NR₁₂- bivalent ; Z₁ est -O-, -O-(O)C-, -C(O)-O- ou -O-C(O)-O-; R₃ est H, C₁-C₁₂-alkyle, C₁-C₁₂-alcoxy ou N-C₁-C₁₂-alkylamino ; R₄ et R₅ sont, indépendamment l'un de l'autre, H, C₁-C₈-alkyle linéaire ou ramifié, C₁-C₈-hydroxyalkyle ou C₆-C₁₀-aryle, ou les groupes R₄-(O)_{b1}- et R₄-(O)_{b2}- forment ensemble -(CH₂)_{c}- dans lequel c est nombre entier de 3 à 5, ou les groupes R₄-(O)_{b1}-, R₄-(O)_{b2}- et R₅-(O₁)_{b3} forment ensemble un radical de formule 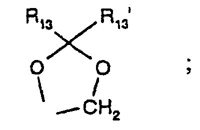 R₂ est une liaison directe ou C₁-C₈-alkylène linéaire ou ramifié non substitué ou substitué par -OH et/ou non interrompu ou interrompu par un ou plusieurs groupes -O-, -O-C(O)- ou -O-C(O)-O- ; R₁ est C₃-C₁₈-alkylène ramifié, C₆-C₁₀-arylène substitué ou non substitué par C₁-C₄-alkyle ou C₁-C₄-alcoxy, ou C₇-C₁₈-aralkylène substitué ou non substitué par C₁-C₄-alkyle ou C₁-C₄-alcoxy, ou C₃-C₈-cycloalkylène substitué ou non substitué ou C₁-C₄-alkyle ou C₁-C₄-alcoxy, ou C₃-C₈-cycloalkylène substitué ou non substitué par C₁-C₄-alkyle ou C₁-C₄-alcoxy, ou C_{y}H_{2y}-(C₃-C₈-cycloalkylène)-C_{y}H_{2y}- non substitué ou substitué par C₁-C₄-alkyle ou C₁-C₄-alcoxy dans lesquels y est un nombre entier de 1 à 6 ; R₆, indépendamment, possède les mêmes significations que R₁ ou est C₃-C₁₈-alkylène ; R₁₂ est C₁-C₆-alkyle
linéaire ou ramifié ; T est -O-, -NH-, -S-, C₁-C₈-alkylène ou 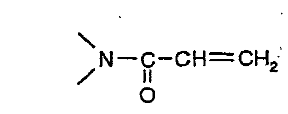 bivalent ; Z₂ est une liaison directe ou -O-(CH₂)_{d}- ou - (OCH₂CH₂)_{d}- dans lesquels d est un nombre entier de 1 à 6 et le groupe terminal CH₂ de chacun est attaché au T adjacent dans la formule (3c) ; R₈ est C₁-C₈-alkyle, C₂-C₈-alkényle ou C₆-C₁₀-aryl-C₁-C₈-alkyle linéaire ou ramifié ; R₉, indépendamment de R₈, possède la même signification que R₈ ou est C₆-C₁₀-aryle, ou R₈ et R₉ forment ensemble -(CH₂)ₑ- dans lequel e est un nombre entier de 2 à 6 ; R₁₀ et R₁₁ sont chacun, indépendamment l'un de l'autre, C₁-C₈-alkyle linéaire ou ramifié qui peut être substitué par C₁-C₄-alcoxy, ou C₆-C₁₀-aryl-C₁-C₈-alkyle ou C₂-C₈-alkényle ; ou R₁₀ et R₁₁ forment -(CH₂)_{f1}-Z₃-(CH₂)_{f2}- dans lequel Z₃ est une liaison directe, -O-, -S-ou -NR₇- et R₇ est H ou C₁-C₈-alkyle et f1 et f2 sont, indépendamment l'un de l'autre, un nombre entier de 2 à 4 ; R₁₃ et R₁₃' sont, indépendamment l'un de l'autre, H, C₁-C₈-alkyle, C₃-C₈-cycloalkyle, benzyle ou phényle ; et a, a1, b1, b2 et b3 sont, indépendamment les uns des autres, 0 ou 1 ; à condition que b1 et b2 soient tous deux 0 lorsque R₁₅ est H ; que le total de (b1+b2+b3) ne soit pas supérieur à 2 ; et que a soit 0 lorsque R₁₂ est une liaison directe.

8. Procédé selon l'une des revendications 1 à 7, **caractérisé en ce qu'**un macromonomère de formule 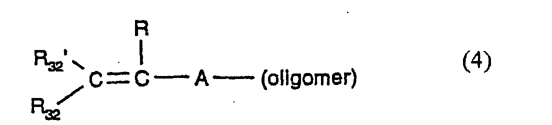 est utilisé à l'étape (c),
dans laquelle R₃₂ est hydrogène, C₁-C₆-alkyle ou un radical -COOR'- ;
R, R' et R₃₂' sont, indépendamment les uns des autres, hydrogène ou C₁-C₆-alkyle ;
A est une liaison directe ou est un radical de formule
-C(O)-(A₁)ₙ-X- (5a);
ou
- (A₂)ₘ-NH-C(O)-X- (5b);
ou
-(A₂)ₘ-X-C(O)- (5c);
ou
-C(O)-NH-C(O)-X- (5d);
ou
-C(O)-X₁-(alk*)-X-C(O)- (5e);
ou
A et R₃₂, de pair avec la liaison double adjacente, sont un radical de formule 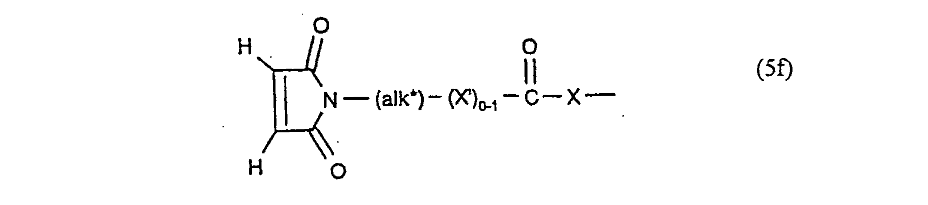 A₁ est -O-C₂-C₁₂-alkylène non substitué ou substitué par hydroxy, ou est - O-C₂-C₁₂-alkylène-NH-C(O)- ou - O-C₂-C₁₂-alkylène-O-C(O)-NH-R₃₃-NH-C(O)- ou -NH-(Alk*)-C(O)-, dans lesquels (Alk*) est C₁-C₆-alkylène et R₃₃ est C₁-C₁₈-alkylène linéaire ou ramifié ou C₆-C₁₀-arylène non substitué ou substitué par C₁-C₄-alkyl- ou C₁-C₄-alcoxy, C₇-C₁₈-aralkylène, C₆-C₁₀-arylène-C₁-C₂-alkylène-C₆-C₁₀-arylène, C₃-C₈-cycloalkylène, C₃-C₈-cycloalkylène-C₁-C₆-alkylène, C₃-C₈-cycloalkylène-C₁-C₂-alkylène-C₃-C₈-cycloalkylène ou C₁-C₆-alkylène-C₃-C₈-cycloalkylène-C₁-C₆-alkylène ;
A₂ est C₁-C₈-alkylène ; phénylène ou benzylène ;
m et n sont, indépendamment l'un de l'autre, un nombre égal à 0 ou 1 ;
X, X₁ et X' sont, indépendamment les uns des autres, un groupe bivalent -O-ou -NR"- dans lequel R" est hydrogène ou C₁-C₆-alkyle ;
(alk*) est C₂-C₁₂-alkylène ;
et (oligomère) désigne
i) le radical d'un télomère de formule 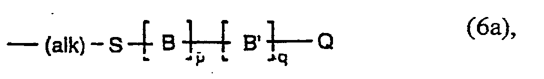 dans laquelle (alk) est C₂-C₁₂-alkylène,
Q est un groupe monovalent pouvant agir en tant que terminateur de réaction de polymérisation en chaîne,
p et q sont, indépendamment l'un de l'autre, un nombre entier de 0 à 350, dans lequel la somme de (p+q) est à un nombre entier de 2 à 350,
et B et B' sont, indépendamment l'un de l'autre, un radical 1,2-éthylène dérivant d'un monomère vinylique copolymérisable en remplaçant la liaison double vinylique par une simple liaison, au moins un des radicaux B et B' étant substitué par un substituant hydrophile ; ou
ii) le radical d'un oligomère de formule 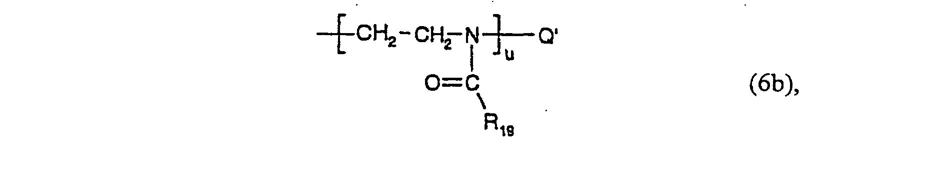 dans laquelle R₁₉ est hydrogène ou non substitué ou hydroxy-C₁-C₁₂-alkyle substitué, u est un nombre entier de 2 à 250 et Q' est un radical d'un initiateur de polymérisation ; ou
iii) le radical de formule 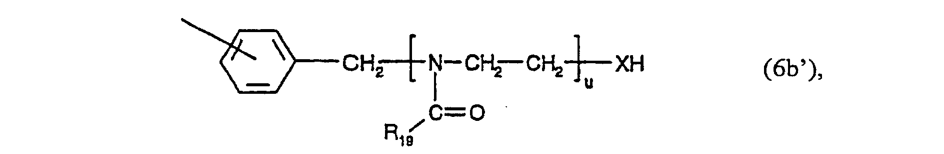 dans laquelle R₁₉, X et u sont tels que définis ci-dessus, ou
iv) le radical d'un oligomère de formule 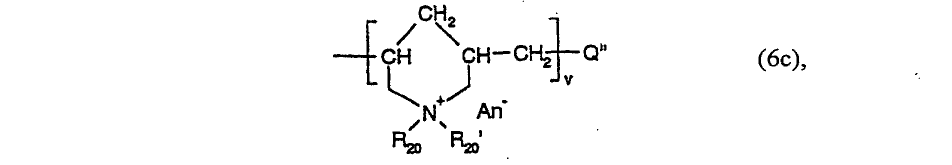 dans laquelle R₂₀ et R₂₀' sont, indépendamment l'un de l'autre, C₁-C₄-alkyle, An⁻ est un anion, v est un nombre entier de 2 à 250 et Q" est un groupe monovalent pouvant agir en tant que terminateur de réaction de polymérisation en chaîne ; ou
v) le radical d'un oligopeptide de formule
- (CHR₂₁-C(O)-NH)ₜ-CHR₂₁-COOH (6d)
ou
- CHR₂₁-(NH-C(O)-CHR₂₁)ₜ-NH₂ (6d')
dans lesquelles R₂₁ est hydrogène ou C₁-C₄-alkyle non substitué ou substitué par hydroxy, carboxy, carbamoyle, amino, phényle, o-, m- ou p-hydroxyphényle, imidazolyle, indolyle ou un radical -NH-C-(=NH)-NH₂ et t est un nombre entier de 2 à 250, ou le radical d'un oligopeptide basé sur la proline ou l'hydroxyproline ; ou
(ii) le radical d'un polyalkylène oxyde de formule
- (alk**-O)_{z}-[CH₂-CH₂-O]ᵣ-[CH₂-CH(CH₃)-O]ₛ-R₃₄ (6e),
dans laquelle R₃₄ est hydrogène ou C₁-C₂₄-alkyle, (alk**) est C₂-C₄-alkylène, z est 0 ou 1, r et s sont, indépendamment l'un de l'autre, un nombre entier de 0 à 250 et la somme de (r+s) va de 2 et 250; ou
(iii) le radical d'un oligosaccharide ;
à condition que
A ne soit pas une liaison directe si (oligomère) est un radical de formule (6a) ;
A soit un radical de formule (5a), (5b) ou (5d) ou A et R₃₂, de pair avec la liaison double adjacente, forment un radical de formule (5f) si (oligomère) est un radical de formule (6b), (6c), (6d) ou (6e) ou est le radical d'un oligosaccharide ;
A soit une liaison directe si (oligomère) est un radical de formule (6b') ; et
A soit un radical de formule (5c) ou (5e) si (oligomère) est un radical de formule (6d').

9. Procédé selon la revendication 8, **caractérisé en ce que** R est hydrogène ou méthyle, R₃₂ et R₃₂' sont tous deux hydrogène, A est un radical de formule (5a) et (oligomère) est un radical de formule (6a).

10. Procédé selon la revendication 8 ou 9, **caractérisé en ce que** (oligomère) est un radical de formule 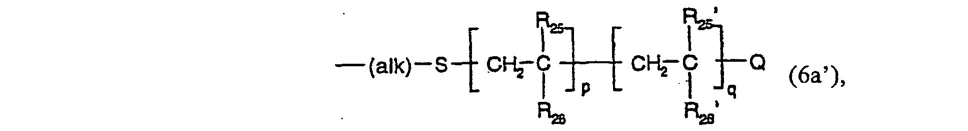 dans laquelle (alk) est C₂-C₄-alkylène, R₂₅ et R₂₅' sont, indépendamment l'un de l'autre, hydrogène ou méthyle, Q est un groupe monovalent pouvant agir en tant que terminateur de réaction de polymérisation en chaîne, p et q sont, indépendamment l'un de l'autre, un nombre entier de 0 à 100 dans lequel la somme de (p+q) est un nombre entier de 5 à 100, et R₂₆ et R₂₆' sont, indépendamment l'un de l'autre, un radical -COOY dans lequel Y est C₁-C₂-alkyle, C₂-C₃-alkyle, substitué par hydroxy, amino ou N,N-di-C₁-C₂-alkyl-amino, ou est un radical -C₂-C₄-alkylène-NH-C(O)-O-G dans lequel -O-G est le radical du tréhalose ; un radical -CO-NY₁Y₂, dans lequel Y₁ et Y₂ sont, indépendamment l'un de l'autre, hydrogène ou C₁-C₂-alkyle, non substitué ou substitué par hydroxy, ou Y₁ et Y₂, de pair avec l'atome d'azote adjacent forment un cycle N-C₁-C₂-alkylpipérazino ou morpholino ; un radical hétérocyclique sélectionné à partir du groupe formé par N-pyrrolidonyle, 2- ou 4-pyridinyle, 2-méthylpyridin-5-yle, 2-, 3- ou 4-hydroxypyridinyle, N-ε-caprolactamyle, N-imidazolyle, 2-méthylimidazol-1-yle, N-morpholinyle et 4-N-méthylpipérazin-1-yle ; -COOH; -SO₃H; o-, m- ou p-sulfophényle ; o-, m- ou p-sulfométhylphényle; un radical -CONY₅Y₆ dans lequel Y₅ est C₂-C₄-alkyle substitué par sulfo, et Y₆ est hydrogène ; C₁-C₄-alkyle substitué par -NR₂₃R₂₃'R₂₃"⁺An⁻ dans lequel R₂₃, R₂₃' et R₂₃" sont, indépendamment les uns des autres, hydrogène ou C₁-C₄-alkyle et An⁻ est un anion ; un -C(O)OY₇ dans lequel Y₇ est C₂-C₄-alkyle substitué par -NR₂₃R₂₃'R₂₃"⁺An⁻ et est par la suite non substitué ou substitué par hydroxy, dans lequel R₂₃, R₂₃', R₂₃" et An⁻ sont tels que définis ; et un radical -C(O)O-CH₂-CH(OY₈)-CH₂-O-PO₂-(CH₂)₂-N(CH₃)₃⁺ dans lequel Y₈ est hydrogène ou le radical acyle d'un acide gras supérieur.

11. Procédé selon l'une des revendications 1 à 10, **caractérisé en ce que** lors de l'étape (c), un macromonomère de formule 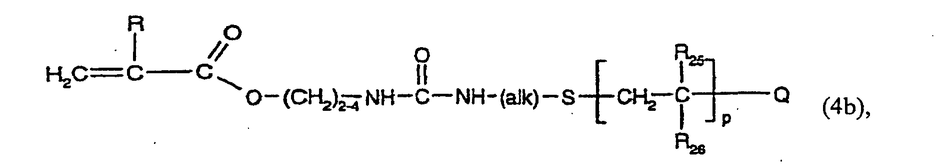 est utilisé, dans laquelle R est hydrogène ou méthyle, (alk) est C₂-C₄-alkylène, R₂₅ est hydrogène ou méthyle, p est un nombre entier de 5 à 50, Q est un groupe monovalent pouvant agir en tant que terminateur de réaction de polymérisation en chaîne et R₂₆ est un radical -CONH₂, - CON(CH₃)₂ ou

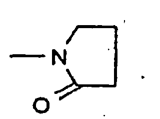

12. Procédé selon la revendication 1 comprenant les étapes suivantes :
a. application sur la surface d'un matériau d'un composé de formule 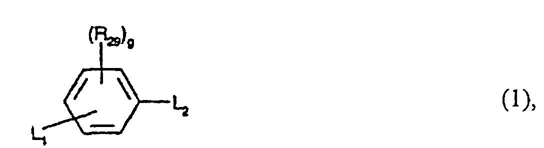 dans laquelle g est 0 ou 1, R₂₉ est méthyle, méthoxy, hydroxy ou nitro, L₁ est le radical azide -N₃, et L₂ est amino, carboxy, un dérivé carboxy, isocyanato ou isothiocyanato ;
b. fixation du composé de formule (1) sur la surface du matériau par rayonnement ;
c. mise en réaction de la surface ainsi modifiée avec un initiateur fonctionnel de polymérisation ayant un groupe fonctionnel coréagissant avec L₂ ; et
d. application d'un macromonomère hydrophile de formule 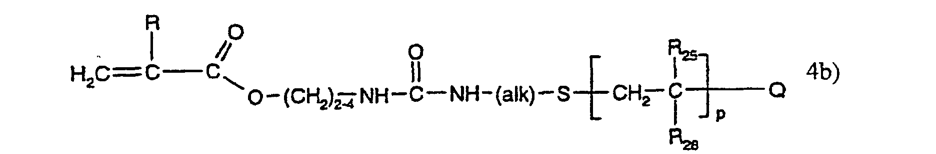 dans laquelle R et R₂₅ sont, indépendamment l'un de l'autre, hydrogène ou méthyle, (alk) est 1,2-éthylène, R₂₆ est -CONH₂, -CON(CH₃)₂ ou 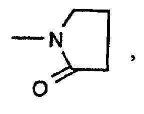 p est un nombre entier de 5 à 250 et Q est un groupe monovalent pouvant agir en tant que terminateur de réaction de polymérisation en chaîne, sur la surface du matériau brut obtenu conformément à l'étape (b) et polymérisation dudit macromonomère, procurant ainsi une surface de recouvrement sur la surface du matériau.

13. Procédé selon l'une des revendications 1 à 12, **caractérisé en ce que** la surface du matériau est la surface d'un dispositif biomédical plus particulièrement, d'une lentille de contact, d'une lentille intraoculaire ou d'une cornée artificielle.
